# EUROPEAN PATENT APPLICATION

(11) **EP 2 319 499 A1**
(43) Date of publication of application: **11.05.2011**
(21) Application number: 10180425.0
(22) Date of filing: 27.01.2006
(51) Int. Cl.: A61K 9/16, A61K 9/20

(54) **Alcohol resistant dosage forms**

(30) Priority: 28.01.2005 GB 0501638; 11.02.2005 WO PCT/GB2005/050014; 12.04.2005 US 670506 P; 26.10.2005 US 730339 P
(62) Divisional of application: 06703915.6
(71) Applicant: EURO-CELTIQUE S.A., 1653 Luxembourg (LU)
(72) Inventor: Mannion, Richard, O., Furlong, PA 18925 (US); Mckenna William, H., Yonkers, NY 10704 (US); O'Donnell, Edward, P., Basking Ridge, NJ 07920 (US); Danagher, Helen, Kathleen, Cambridge, Cambridgeshire CB4 0GW (GB); Hayes, Geoffrey, Gerard, Cambridge, Cambridgeshire CB4 0GW (GB); Mohammad, Hassan, Cambridge, Cambridgeshire CB4 0GW (GB); Prater, Derek, Allan, Cambridge, Cambridgeshire CB4 0GW (GB); Tamber, Harjit, Cambridge, Cambridgeshire CB4 0GW (GB); Walden, Malcolm, Cambridge, Cambridgeshire CB4 0GW (GB); Whitelock, Steve, Cambridge, Cambridgeshire CB4 0GW (GB); Fleischer, Wolfgang, 55218, Ingelheim (DE); Hahn, Udo, 56412, Nentershausen (DE); Spitzley, Christof, 65627, Elbgrund (DE); Leuner,, Christian, 60439, Frankfurt (DE)
(74) Representative: Glas, Holger

(57) **Abstract**

Opioid controlled release formulations resistant to alcohol extraction of the opioid.

## Description

This application claims the benefit of GB patent application no. 0501638.1, filed on January 28, 2045, of PCT patent application no. PCT/GB2005/050014 filed on February 11, 2005, of US provisional application no. 60/670,506, filed on April 12, 2005 and of US provisional application no. 60/730,339, filed on October 26, 2005.

### TECHNICAL FIELD OF THE INVENTION

The present invention relates to controlled release formulations resistant to alcohol extraction, in particular opioid controlled release formulations resistant to alcohol extraction.

### BACKGROUND OF THE INVENTION

Pharmaceutical products are sometimes the subject of abuse. For example, a particular dose of opioid agonist may be more potent when administered parenterally as compared to the same dose administered orally. Some formulations can be tampered with to provide the opioid agonist contained therein for illicit use. Controlled release opioid agonist formulations are sometimes crushed, or subject to extraction with solvents (e.g., ethanol) by drug abusers to provide the opioid contained therein for immediate release upon oral or parenteral administration.

Controlled release opioid agonist dosage forms which can liberate a portion of the opioid upon exposure to ethanol, can also result in a patient receiving the dose more rapidly than intended if a patient disregards instructions for use and concomitantly uses alcohol with the dosage form.

Purdue Pharma L.P. currently markets sustained-release oxycodone in dosage forms containing 10, 20, 40 and 80 mg oxycodone hydrochloride under the tradename OxyContin.

U.S. Patent Nos. 5,266,331; 5,508,042; 5,549,912 and 5,656,295 disclose sustained release oxycodone formulations.

Purdue Pharma L.P. is the NDA holder of sustained-release hydromorphone in dosage forms containing 12, 16, 24 and 32 mg hydromorphone hydrochloride under the tradename Palladone®.

During the development of Palladone®, in-vitro extraction and dissolution studies indicated that exposure of the formulation to ethanol increased the release of hydromorphone, as compared to release in water. Subsequent pharmacokinetic studies in healthy subjects have shown that the concomitant intake of ethanol with Palladone® Capsules can result in the rapid release and absorption of hydromorphone from the formulation.

U.S. Patent Nos. 5,958,452; 5,965,161; 5,968,551; 6,294,195; 6,335,033; 6,706,281; and 6,743,442 disclose sustained release hydromorphone formulations.

U.S. Patent Publication Nos. 2003/0118641 and 2005/0163856 to Maloney et al. describe an opioid formulation which employs an ion exchange resin in conjunction with a hydrophobic matrix that is purportedly resistant to extraction of the opioid with commonly available solvents.

U.S Patent Publication No. 2004/0052731 to Hirsh et al. describes a pharmaceutical composition which can purportedly be used to reduce the likelihood of improper administration of drugs.

There continues to exist a need in the art for an oral dosage form comprising an opioid agonist with reduced opioid release upon exposure to alcohol.

All references cited herein, including the foregoing patents, patent applications and priority documents, are hereby incorporated by reference in their entireties.

### OBJECTS AND SUMMARY OF THE INVENTION

It is an object of certain embodiments of the present invention to provide an oral controlled release dosage form comprising an opioid analgesic which is resistant to common extraction methods, in particular with ethanolic solutions, intended to liberate the opioid analgesic for illicit use.

It is an object of certain embodiments of the present invention to provide an oral controlled release dosage form comprising an opioid analgesic which is resistant to the release of the opioid analgesic when concomitantly used with alcohol.

It is an object of certain embodiments of the present invention to provide an oral controlled release dosage form comprising an opioid analgesic which has increased hardness and is resistant to crushing.

In certain embodiments, the present invention is directed to a controlled release dosage form comprising an opioid analgesic and a controlled release material; wherein the ratio of the amount of opioid analgesic released after 1 hour of in-vitro dissolution of the dosage form in 500 ml and/or 900 mlof Simulated Gastric Fluid with 20% ethanol using a USP Apparatus I (basket) apparatus at 100 rpm at 37 degrees C° to the amount of opioid analgesic released after 1 hour in-vitro dissolution of the dosage form in 500 ml and/or 900 ml of Simulated Gastric Fluid with 0% ethanol using a USP Apparatus I (basket) apparatus at 100 rpm at 37 degrees C° is 2:1 or less or less than about 2:1; 1.5:1 or less or less than about 1.5:1; or 1:1 or less or less than about 1:1. In certain such embodiments, the lower limit of this ratio is 0.5:1 or 1:1.

In certain embodiments, the present invention is directed to a controlled release dosage form comprising an opioid analgesic and a controlled release material; wherein the ratio of the amount of opioid analgesic released after 1 hour of in-vitro dissolution of the dosage form in 500 ml and/or 900 ml of Simulated Gastric Fluid with 30% ethanol using a USP Apparatus I (basket) apparatus at 100 rpm at 37 degrees C° to the amount of the opioid analgesic released after 1 hour of in-vitro dissolution of the dosage form in 500 ml and/or 900 ml of Simulated Gastric Fluid with 0% ethanol using a USP Apparatus I (basket) apparatus at 100 rpm at 37 degrees C° is 4:1 or less or less than about 4:1; 3:1 or less or less than about 3:1; or 2:1 or less or less than about 2:1. In certain such embodiments, the lower limit of this ratio is 0.5:1 or 1:1; or 1.7:1.

In certain embodiments, the present invention is directed to a controlled release dosage form comprising an opioid analgesic and a controlled release material; wherein the ratio of the amount of opioid analgesic released after 1 hour of in-vitro dissolution of the dosage form in 500 ml and/or 900 ml of Simulated Gastric Fluid with 40% ethanol using a USP Apparatus I (basket) apparatus at 100 rpm at 37 degrees C° to the amount of the opioid analgesic released after 1 hour of in-vitro dissolution of the dosage form in 500 ml and/or 900 ml of Simulated Gastric Fluid with 0% ethanol using a USP Apparatus I (basket) apparatus at 100 rpm at 37 degrees C° is 5:1 or less or less than about 5:1; 4:1 or less or less than about 4:1; or 3:1 or less or less than about 3:1. In certain such embodiments, the lower limit of this ratio is 0.5:1 or 1:1; or 2.6:1.

In certain embodiments, the present invention is directed to a controlled release dosage form comprising a plurality of matrices comprising a therapeutically effective amount of hydromorphone or a pharmaceutically acceptable salt thereof, dispersed in a controlled release material; wherein the ratio of the amount of hydromorphone or a pharmaceutically acceptable salt thereof released after 1 hour of in-vitro dissolution of the dosage form in 500 ml and/or 900 ml of Simulated Gastric Fluid with 20% ethanol using a USP Apparatus I (basket) apparatus at 100 rpm at 37 degrees C° to the amount of hydromorphone or a pharmaceutically acceptable salt thereof released after 1 hour of in-vitro dissolution of the dosage form in 500 ml and/or 900 ml of Simulated Gastric Fluid with 0% ethanol using a USP Apparatus I (basket) apparatus at 100 rpm at 37 degrees C° is less than about 2:1. In more detail: In certain such embodiments, the present invention is directed to a controlled release dosage form comprising a plurality of extruded matrices comprising a therapeutically effective amount of hydromorphone or a pharmaceutically acceptable salt thereof, dispersed in an alkylcellulose. In certain such embodiments, the present invention is directed to a controlled release dosage form comprising a plurality of extruded matrices comprising a therapeutically effective amount of hydromorphone or a pharmaceutically acceptable salt thereof, dispersed in an ethylcellulose. In certain such embodiments, the present invention is directed to a controlled release dosage form comprising a plurality of extruded matrices comprising a therapeutically effective amount of hydromorphone or a pharmaceutically acceptable salt thereof, dispersed in an alkylcellulose, the alkylcellulose being at least 50 %, w/w of the matrices. In certain such embodiments, the present invention is directed to a controlled release dosage form comprising a plurality of extruded matrices consisting essentially of hydromorphone or a pharmaceutically acceptable salt thereof, dispersed in an alkylcellulose. In certain such embodiments, the present invention is directed to a controlled release dosage form comprising a plurality of extruded matrices consisting essentially of hydromorphone or a pharmaceutically acceptable salt thereof, dispersed in an alkylcellulose, an optional binder, and an optional plasticizer. In certain such embodiments, the present invention is directed to a controlled release dosage form comprising a plurality of extruded matrices comprising hydromorphone or a pharmaceutically acceptable salt thereof, dispersed in an alkylcellulose, wherein the matrices do not comprise an acrylic polymer.

In certain embodiments, the present invention is directed to a controlled release dosage form comprising a matrix comprising a pharmaceutically acceptable salt of an opioid analgesic in a controlled release material; wherein less than 25% of the opioid salt is released after 1 hour of in-vitro dissolution of the dosage form in 500ml and/or 900 ml of Simulated Gastric Fluid with 20% ethanol using a USP Apparatus I (basket) apparatus at 100 rpm at 37 degrees C°. In more detail: In certain such embodiments, the present invention is directed to a controlled release dosage form comprising a plurality of matrices comprising a pharmaceutically acceptable salt of an opioid analgesic in a controlled release material. In certain such embodiments, the present invention is directed to a controlled release dosage form comprising a matrix comprising a pharmaceutically acceptable salt of an opioid analgesic in a pharmaceutically acceptable excipient; and a layer comprising a controlled release material disposed about the matrix, e.g. In certain such embodiments, the present invention is directed to a controlled release dosage form comprising a plurality of matrices comprising a pharmaceutically acceptable salt of an opioid analgesic in a pharmaceutically acceptable excipient; and a layer comprising a controlled release material disposed about each of the matrices.

In certain embodiments the present invention is directed to the use of a sparingly water permeable thermoplastic polymer or a hydrophobic polymer as controlled release matrix material in the manufacture of an opioid controlled release matrix formulation to impart resistance to alcohol extraction of the opioid, wherein said formulation having the sparingly water permeable thermoplastic polymer or hydrophobic polymer as controlled release matrix material releases less opioid in an alcohol extraction test compared to the same formulation but with the sparingly water permeable thermoplastic polymer or hydrophobic polymer substituted entirely or partly by other matrix materials.

In certain embodiments the present invention is directed to the use of a sparingly water permeable thermoplastic polymer as controlled release matrix material in the manufacture of an opioid controlled release matrix formulation to impart resistance to alcohol extraction, wherein said formulation after 15 minutes shaking in 40 % ethanol at room temperature using a Stuart Scientific Flask Shaker Model SF1 set at 500 to 600 oscillations per minute releases less than 35 % of opioid. In certain such embodiments said formulation releases less than 30 %, more preferred less than 25 % of opioid salt, or from 15 to 25 % opioid salt.

In certain embodiments the present invention is directed to the use of a hydrophobic material polymer as controlled release matrix material in the manufacture of an opioid controlled release matrix formulation to impart resistance to alcohol extraction, wherein less than 25 % of the opioid is released after 1 hour of in-vitro dissolution of a dosage form comprising said formulation in 500 ml and/or 900 ml of Simulated Gastric Fluid with 20 % ethanol using USP Apparatus I (basket) operating at 100 rpm at 37 °C.

In certain embodiments the present invention is directed to the use of a hydrophobic material as controlled release matrix material in the manufacture of an opioid controlled release matrix formulation to impart resistance to alcohol extraction, wherein the ratio of the amount of opioid released after 1 hour of in-vitro dissolution of the dosage form comprising said formulation in 900 and/or 500 ml of Simulated Gastric Fluid with 20 % ethanol using a USP Apparatus I (basket) apparatus at 100 rpm at 37 °C, to the amount of opioid released after 1 hour of in-vitro dissolution of the dosage form comprising said formulation in 900 and/or 500 ml of Simulated Gastric Fluid with 0 % ethanol using an USP Apparatus I (basket) apparatus at 100 rpm at 37 °C, is less than about 2:1.

In certain embodiments, the present invention is directed to a method of treating pain comprising administering to a patient in need thereof a dosage form as disclosed herein. In certain embodiments, the present invention is directed to a method of deterring abuse of an opioid agonist comprising preparing a dosage form as disclosed herein.

The formulations disclosed herein are intended to release the drug over an extended period of time to provide a therapeutic effect. In certain embodiments, the controlled release formulations provide a at least a 12 hour or 24 hour therapeutic effect.

The term "controlled release" as it applies to an opioid agonist is defined for purposes of the present invention as the release of the opioid from the formulation at a rate which will provide a longer duration of action than a single dose of the normal (i.e., immediate release) formulation. For example, an immediate release oral formulation may release the drug over a 1-hour interval, compared to a controlled release oral formulation which may release the drug over a 4 to 24 hour interval.

For purposes of the present invention, the term "opioid analgesic" is interchangeable with the term "opioid" and includes one agonist or a combination of more than one opioid agonist, and also includes the use of a mixed agonist-antagonist; a partial agonist and combinations of an opioid agonist and an opioid antagonist, wherein the combination provides an analgesic effect, stereoisomers thereof; an ether or ester thereof; or a mixture of any of the foregoing. With respect to certain embodiments of the present invention, the term "opioid agonist" is interchangeable with the term "opioid analgesic" and includes one agonist or a combination of more than one opioid agonist, and also includes the use of a mixed agonist-antagonist; a partial agonist; stereoisomers thereof; an ether or ester thereof; or a mixture of any of the foregoing.

The present invention disclosed herein is meant to encompass the use of any pharmaceutically acceptable salt of the opioid. The term "opioid salt" refers to a pharmaceutically acceptable salt of the opioid. Any embodiment of the invention referring to opioid is also meant to refer to opioid salt.

Pharmaceutically acceptable salts include, but are not limited to, metal salts such as sodium salt, potassium salt, secium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate, and the like; amino acid salts such as arginate, asparginate, glutamate and the like.

The opioids used according to the present invention may contain one or more asymmetric centers and may give rise to enantiomers, diastereomers, or other stereoisomeric forms. The present invention is also meant to encompass the use of all such possible forms as well as their racemic and resolved forms and mixtures thereof. When the compounds described herein contain olefinic double bonds or other centers of geometric asymmetry, it is intended to include both E and Z geometric isomers. All tautomers are intended to be encompassed by the present invention as well.

In certain embodiments, the matrix or plurality of matrices of the dosage form disclosed herein consist essentially of an opioid analgesic dispersed in an alkylcellulose; an optional binder, and an optional plasticizer.

In certain embodiments, the dosage form as disclosed herein does not comprise an acrylic polymer. In certain embodiments, the matrix or plurality of matrices of the dosage form disclosed herein do not comprise an acrylic polymer.

As used herein, the term "stereoisomers" is a general term for all isomers of individual molecules that differ only in the orientation of their atoms is space. It includes enantiomers and isomers of compounds with more than one chiral center that are not mirror images of one another (diastereomers).

The term "chiral center" refers to a carbon atom to which four different groups are attached.

The term "enantiomer" or "enantiomeric" refers to a molecule that is nonsuperimposeable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule.

The term "layer" means a material disposed about a substrate (which can include itself and one or more optional intermediate layers such e.g., a seal coat), which can be applied, e.g., as a coating. Layering of substrates can be performed by procedures known in the art including, e.g., spray coating, dipping or enrobing.

The term "disposed about" means that the layer material disposed about the particle covers at least a portion of the particle, with or without an intermediate layer or layers between the substance and the particle. In certain embodiments, the material covers an average of at least 50% of the surface area of the particle. In certain other embodiments, the material completely covers the particle.

The term "resistance to alcohol extraction" in the broadest sense refers to the ability of a formulation to release less opioid when subjected to a solution comprising ethanol than a comparative formulation, notwithstanding the fact that "resistance to alcohol extraction" can be alternatively or further defined with respect to specific embodiments of the invention. Within the meaning of the present invention resistance to alcohol extraction can be tested and defined by various "alcohol extraction tests" which involve subjecting the formulation to a solution comprising ethanol as described herein.

The term "controlled release matrix formulation" refers to the composition including the controlled release materials and the opioid. Unless specifically indicated the term "controlled release matrix formulation" refers to said formulation in intact form.

The term "controlled release dosage form" refers to the administration form comprising the opioid in controlled release form as e.g. in form of the "controlled release matrix formulation" or in any other controlled release form as referred to herein. Unless specifically indicated the term "controlled release dosage form" refers to said dosage form in intact form.. The dosage form can e.g. be a tablet comprising the compressed controlled release matrix formulation or a capsule comprising the controlled release matrix formulation in the form of multi particulates..

Resistance to alcohol extraction can e.g. be tested by subjecting the formulation to Simulated Gastric Fluid (SGF) with 20% ethanol. A typical manner in order to obtain "900 ml of Simulated Gastric Fluid (SGF) with 20% ethanol" is by mixing 800 ml of SGF with 210 ml of 95% ethanol/water (which provides 200 ml ethanol) and taking 900 ml of the mixture. The effect of the additional 10 ml of water from the 95% ethanol will be minimal in the percentages of SGF and ethanol in the 900 ml mixture. Resistance to alcohol extraction can also be tested using an aqueous solution comprising 40% ethanol.

### BRIEF DESCRIPTION OF THE DRAWINGS

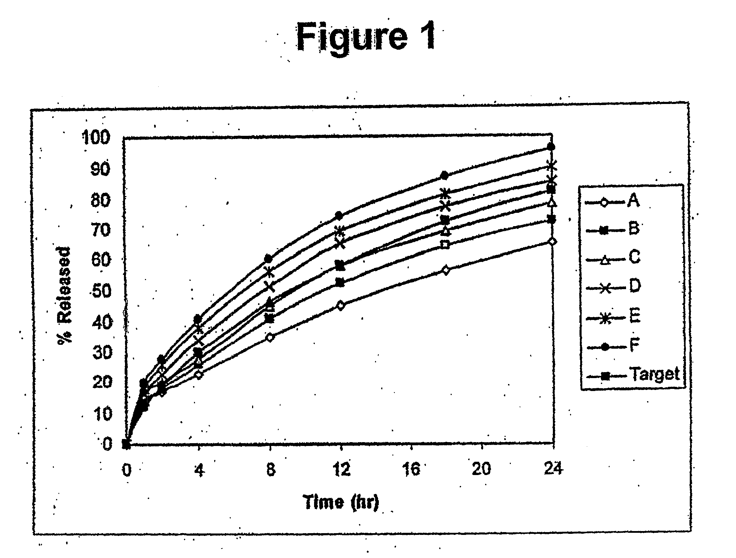

Figure 1 depicts the in-vitro dissolution results of compositions A-F of Example 5.
Figure 2 and 3 depict the in-vitro dissolution results of Example 9.
Figure 4 depicts the crushing test results using a Pill Crusher or Spoons of Example 14
Figure 5 depicts the crushing test results using Mortar and Pestle of Example 14
Figure 6 depicts the alcohol extraction test results of Example 14.
Figure 7 depicts the alcohol extraction test results of Examples 15 to 21 described in Example 25.
Figure 8 depicts the dissolution profiles in Simulated Gastric Fluid with 40% alcohol of Examples 15 to 21 described in Example 25
Figure 9 depicts the dissolution profiles in Simulated Gastric Fluid of Examples 15 to 20 described in Example 25
Figure 10 depicts the dissolution profiles in Simulated Gastric Fluid 40% alcohol of Examples 22 to 24 described in Example 25
Figure 11 depicts the dissolution profiles in Simulated Gastric Fluid of Examples 22 to 24 described in Example 25

### DETAILED DESCRIPTION

Drug abusers sometimes try to achieve euphoric effects by manipulating drug formulations to quicken the onset.

The most rudimentary way of accomplishing this is by crushing the dosage form into a fine powder in an attempt to make the active ingredient more available. Oral abusers chew and/ or swallow the material, and nasal abusers crush the formulations for snorting.

For parenteral or intravenous tampering, crushed material is sometimes dissolved in water with heating and filtered into a syringe for injection.

In addition to the aforementioned "direct tampering" techniques, more determined abusers can also use various kinds of "kitchen chemistry" in an attempt to completely isolate the active ingredient from a formulation matrix. One method involves one-step extractions into commonly available media such as water or ethanol and mixtures thereof.

In certain embodiments, the present invention is directed to a controlled release dosage form comprising a matrix comprising a pharmaceutically acceptable salt of an opioid analgesic in a controlled release material; wherein less than 25%, or less than 20% of the opioid salt is released after 1 hour of in-vitro dissolution of the dosage form in 500 ml and/or 900 ml of Simulated Gastric Fluid with 20% ethanol using a USP Apparatus I (basket) apparatus at 100 rpm at 37 degrees C°. In certain such embodiments, at least 5%, or 10% opioid analgesic is released under these dissolution conditions. In more detail: In certain such embodiments, the present invention is directed to a controlled release dosage form comprising a plurality of matrices comprising a pharmaceutically acceptable salt of an opioid analgesic in a controlled release material. In certain such embodiments, the present invention is directed to a controlled release dosage form comprising a matrix comprising a pharmaceutically acceptable salt of an opioid analgesic in a pharmaceutically acceptable excipient; and a layer comprising a controlled release material disposed about the matrix. In certain such embodiments, the present invention is directed to a controlled release dosage form comprising a plurality of matrices comprising a pharmaceutically acceptable salt of an opioid analgesic in a pharmaceutically acceptable excipient; and a layer comprising a controlled release material disposed about each of the matrices.

In certain such embodiments the controlled release dosage form comprises a controlled release matrix formulation which does not contain more than 15% (by wt), preferably which does not contain more than 20 % (by wt) C₁₂ to C₃₆ aliphatic alcohol selected from the group consisting of stearyl alcohol, cetyl alcohol and cetostearyl alcohol.

In certain embodiments, the present invention is directed to a controlled release dosage form comprising an opioid analgesic and a controlled release material; wherein the ratio of the amount of opioid analgesic released after 1 hour of in-vitro dissolution of the dosage form in 500 ml and/or 900 ml of Simulated Gastric Fluid with 20% ethanol using a USP Apparatus I (basket) apparatus at 100 rpm at 37 degrees C° to the amount of opioid analgesic released after 1 hour in-vitro dissolution of the dosage form in 500 ml and/or 900 ml of Simulated Gastric Fluid with 0% ethanol using a USP Apparatus I (basket) apparatus at 100 rpm at 37 degrees C° is 2:1 or less or less than about 2:1; 1.5:1 or less or less than about 1.5:1; or 1:1 or less or less than about 1:1. In certain such embodiments, the lower limit of this ratio is 0.5:1 or 1:1.

In certain such embodiments the controlled release dosage form comprises a controlled release matrix formulation which does not contain more than 15% (by wt), preferably which does not contain more than 20 % (by wt) C₁₂ to C₃₆ aliphatic alcohol selected from the group consisting of stearyl alcohol, cetyl alcohol and cetostearyl alcohol.

In certain embodiments the present invention is directed to the use of a sparingly water permeable thermoplastic polymer as controlled release matrix material in the manufacture of an opioid controlled release matrix formulation to impart resistance to alcohol extraction, wherein said formulation after 15 minutes shaking in 40 % ethanol at room temperature using a Stuart Scientific Flask Shaker Model SF1 set at 500 to 600 oscillations per minute releases less than 35 % of opioid. In certain such embodiments said formulation releases less than 30 %, more preferred less than 25 % of opioid salt, or from 15 to 25 % opioid salt.

In certain embodiments the present invention is directed to the use of a hydrophobic material polymer as controlled release matrix material in the manufacture of an opioid controlled release matrix formulation to impart resistance to alcohol extraction, wherein less than 25 % of the opioid is released after 1 hour of in-vitro dissolution of a dosage form comprising said formulation in 500ml and/or 900 ml of Simulated Gastric Fluid with 20 % ethanol using USP Apparatus I (basket) operating at 100 rpm at 37 °C.

In certain embodiments the present invention is directed to the use of a hydrophobic material as controlled release matrix material in the manufacture of an opioid controlled release matrix formulation to impart resistance to alcohol extraction, wherein the ratio of the amount of opioid released after 1 hour of in-vitro dissolution of the dosage form comprising said formulation in 900 and/or 500 ml of Simulated Gastric Fluid with 20 % ethanol using a USP Apparatus I (basket) apparatus at 100 rpm at 37 °C, to the amount of opioid released after 1 hour of in-vitro dissolution of the dosage form comprising said formulation in 900 and/or 500 ml of Simulated Gastric Fluid with 0 % ethanol using an USP Apparatus I (basket) apparatus at 100 rpm at 37 °C, is less than about 2:1.

In any of the embodiments disclosed herein, the dosage form can comprise a matrix comprising the opioid analgesic and the controlled release material; a plurality of matrices comprising the opioid analgesic and the controlled release material; a matrix comprising the opioid analgesic and a pharmaceutically acceptable excipient and a layer comprising a controlled release material disposed about the matrix; or a plurality of matrices comprising the opioid analgesic and a pharmaceutically acceptable excipient and a layer comprising a controlled release material disposed about each of the matrices. This list is not meant to be exclusive.

In certain embodiments, the dosage form can comprise an opioid analgesic in an osmotic core with a semipermeable membrane surrounding the core. The dosage form can have an optional passageway for osmotic delivery of the opioid analgesic upon administration.

In certain embodiments of the present invention, the controlled release material comprises a hydrophobic material, preferably an alkylcellulose, and most preferably ethylcellulose. In certain embodiments of the present invention, the controlled release material comprises a sparingly water permeable thermoplastic polymer, preferably an alkylcellulose, and most preferably ethylcellulose.

In certain embodiments of the invention the above said hydrophobic material or said sparingly water permeable thermoplastic polymer is used to impart resistance to alcohol extraction as described herein. The embodiments described below provide a more detailed description of the use of said hydrophobic material or said sparingly water permeable thermoplastic polymers to impart resistance to alcohol extraction.

In certain embodiments, the ethylcellulose is present in a weight amount of at least 40%, at least 45%, at least 50%, at least 55% or at least 60% of the matrix or matrices. In other embodiments, the ethylcellulose is present in a weight amount of at most 70%, at most 80% or at most 90% of the matrix or matrices.

In certain embodiments the present invention is directed to the use of alkyl cellulose, preferably ethyl cellulose, in an amount from 5 to 60 % (by wt) of the controlled release matrix formulation, preferably from 10 to 50 % (by wt), most preferably from 20 to 45 % (by wt) of the controlled release matrix formulation.

In certain embodiments the present invention is directed to the use of ethyl cellulose in combination with at least a second controlled release matrix material selected from a polymethacrylate polymer, preferably a neutral water-insoluble poly (ethyl acrylate, methyl methacrylate) copolymer.

In certain embodiments, comprising an alkylcellulose, the controlled release material further comprises a polymethacrylate polymer in a weight amount of at least 5%, at least 10%, at least 15%, at least 20% or at least 25% of the matrix or matrices. In other embodiments, the polymethacrylate polymer is present in a weight amount of at most 30%, or at most 35% of the matrix or matrices.

In certain embodiments comprising alkyl cellulose, preferably ethyl cellulose, the controlled release matrix formulation further comprises a polymethacrylate polymer, preferably a neutral water-insoluble poly(ethyl acrylate, methyl acrylate) copolymer in an amount of 5 % to 66 % (by wt), preferably 15 % to 50 % (by wt), more preferred 20 % to 45 % (by wt) and most preferred 25 % to 45 % (by wt) of the controlled release matrix formulation.

In one aspect, the controlled release pharmaceutical formulation may be obtained or is obtainable by melt extrusion and may include a neutral poly(ethyl acrylate, methyl methacrylate) copolymer and an active ingredient. The rubber-like characteristics of this polymer provide multi particulates which typically are elastic and compressible without breaking, and are preferably resilient.

In one preferred form, the multi particulates may be compressed by hand between two rigid surfaces, for example a coin and a tabletop or between two spoons, without breaking. The multi particulates may be distorted but may not break or shatter and may ideally reassume more or less their original shape.

Rubbery characteristics help impart resistance to tamper. Tamper resistance is of especial importance for products containing opioid analgesics or other active ingredients which are subject to abuse. The tamper resistance of preferred multi particulates of the invention can be demonstrated by shaking a dosage amount of multi particulates in water and/or ethanol, for example 40% ethanol.

When tested in this way, preferred multi particulates will show at least one of the following release characteristics of active agent:
15 minutes shaking in water at room temperature: less than 15, less than 10% release of active agent, preferably less than 7.5% release of active agent, more preferably less than 5% release of active agent, for example 1.5 to 4% release of active agent.
5 minutes standing in water at 50°C followed by 15 minutes shaking at the same temperature: less than 20% release of the active agent, preferably less than 15% release of active agent, more preferably less than 12% release of active agent, for example 4 to 12% release of active agent.
5 minutes standing at 75°C followed by 15 minutes shaking at the same temperature: less than 25% release of active agent, preferably less than 20% release of active agent, more preferably less than 15% release of active agent, for example 10 to 15% release of active agent.
5 minutes standing at 100°C followed by 15 minutes shaking at the same temperature: less than 30'% release of active agent, preferably less than 25% release of active agent, more preferably less than 20% release of active agent, for example 12 to 20% release of active agent.
15 minutes shaking in 40% ethanol at room temperature: less than 35% release of active agent, preferably less than 30% release of active agent, more preferably less than 25% release of active agent, for example 15 to 20% release of active agent.

Alternatively, the tamper resistance of preferred multi particulates of the invention can be demonstrated by subjecting a dosage amount of multi particulates to grinding in a mortar and pestle with 24 rotations of the pestle and the product placed in 900 ml water at 37°C for 45 minutes. The amounts of active agent extracted can then be determined by HPLC and detection UV for instance at 210 nm wavelength.

When tested using this method, preferred multi particulates according to the invention will show the following release characteristics of active agent; less than 12.5% release agent, preferably less than 10% release of active agent, more preferably less than 7.5% release of active agent, for example 2 to 7.5% release of active agent.

In a further method, the tamper resistance of preferred multi particulates of the invention can be demonstrated by crushing a dosage amount of multi particulates between two spoons or in a pill crusher, such as a Pill Pulverizer as sold by Apex Healthcare Products, and then extracting in 2 ml water heated to boiling on a spoon and filtered off. The amounts of active agent extracted can then be determined by HPLC and detection by UV for instance at 210 mm wavelength.

When tested using this method, preferred multi particulates according to the invention will show the following release characteristics of active agent; less than 27.5% release of active agent, preferably less than 15% release of active agent, more preferably less than 5% release of active agent, for example 1 to 5% release of active agent.

For imparting such tamper resistance, the present invention may include the use of a neutral poly(ethyl acrylate, methylacrylate) copolymer in the preparation of a pharmaceutical formulation to provide resistance to tamper. A neutral poly(ethyl acrylate, methyl methacrylate) copolymer may be incorporated with the active ingredient in the formulation.

In certain embodiments of the present invention, the dosage form further comprises a binder in a weight amount of at least 1%, at least 3%, or at least 5% of the matrix or matrices. In other embodiments, the binder is in a weight amount of at most 7%, or at most 10% of the matrix or matrices. In certain embodiments, the binder is a hydroxyalkylcellulose such as hydroxypropylcellulose or hydroxypropylmethylcellulose.

In certain embodiments of the present invention, the dosage form further comprises a plasticizer in a weight amount of at least 3%, at least 5%, at least 15%, or at least 25% of the matrix or matrices. In other embodiments, the plasticizer is in a weight amount of at most 30%, or at most 40% of the matrix or matrices.

In certain embodiments, the plasticizer has a melting point of at least 80° C. This helps to minimize the dissolution of the dosage form in hot water in an attempt to liberate the opioid analgesic contained therein. In certain embodiments, the plasticizer is hydrogenated castor oil.

A hot water extraction test may be performed as follows: Place one dosage unit of each drug product into two separate glass scintillation vials and label the vials 1 and 2. Add 10 mL of extraction solvent to each vial. If specified, place the vials in a water bath set to a specified temperature (50, 75 or 100°C) for 5 minutes. Place both vials on a laboratory wrist-action shaker and remove vial 1 after 15 minutes and vial 2 after 120 minutes. Samples at room temperature are placed directly onto the shaker.

In certain such embodiments the ratio of the weight% amount of the opioid analgesic released at 50°C, 120 minutes shaking, based on the total amount of opioid in the tested controlled release formulation or dosage form , to the weight% amount of opioid analgesic released at RT 120 minutes shaking, based on the total amount of opioid in the tested controlled release formulation or dosage form is 1.2 or less, preferably 1 or less or 0.9 or less.

In certain such embodiments the ratio of the weight% amount of the opioid analgesic released at 75°C, 15 minutes shaking, based on the total amount of opioid in the tested controlled release formulation or dosage form , to the weight% amount of opioid analgesic released at RT 15 minutes shaking, based on the total amount of opioid in the tested controlled release formulation or dosage form is 1.2 or less, preferably 1 or less or 0.9 or less.

In certain such embodiments the ratio of the weight% amount of the opioid analgesic released at 100°C, 15 minutes shaking, based on the total amount of opioid in the tested controlled release formulation or dosage form , to the weight% amount of opioid analgesic released at RT 15 minutes shaking, based on the total amount of opioid in the tested controlled release formulation or dosage form is 1.3 or less, preferably 1.2 or less or 0.9 or less.

In certain such embodiments the ratio of the weight% amount of the opioid analgesic released at 100°C, 120 minutes shaking, based on the total amount of opioid in the tested controlled release formulation or dosage form , to the weight% amount of opioid analgesic released at RT 120 minutes shaking, based on the total amount of opioid in the tested controlled release formulation or dosage form is less than 2, preferably 1.5 or less or 1 or less or 0.9 or less.

In certain embodiments the amount of the alkyl cellulose, preferably ethyl cellulose, is less than 20 % (by wt), preferably less than 15 % (by wt), most preferred less than 10 % (by wt) but more than 5% (by wt) of the controlled release matrix formulation.

In more detail: In such certain embodiments preferably the alkyl cellulose, especially ethyl cellulose, is used in the form of particles or aqueous alkyl cellulose dispersions.

In case of ethyl cellulose particles, the ethyl cellulose has preferably a viscosity in the range of 3 to 110 cP, when measured in a 5 % solution at 25 °C in an Ubbelohde viscosimeter with a solvent of 80 % toluene and 20 % alcohol. Preferably, the viscosity is in the range of 18 to 110 cP and most preferred in the range of 41 - 49 cP. A suitable ethyl cellulose is provided by Dow Chemical Company under the trade name Ethocel ™ Standard 45. An alternative ethyl cellulose is Ethocel^{™} Standard 7.

In case of aqueous ethyl cellulose dispersions, a dispersion of ethyl cellulose 20 cP with dibutyl/sebacate, ammoniumhydroxide, oleic acid and colloidal anhydrous silica is preferred, which is available under the trade name Surlease^{™} E-7-7050.

In certain embodiments the present invention is directed to the use of ethyl cellulose in combination with at least one plasticizer or second controlled release matrix material selected from C₁₂ to C₃₆ aliphatic alcohols and the corresponding aliphatic acids, preferably stearyl alcohol, cetyl alcohol and cetostearyl alcohol and the corresponding stearic and palmitic acids and mixtures thereof, wherein the amount of C₁₂ to C₃₆ aliphatic alcohol or aliphatic acid is preferably at least 5 %, more preferred at least 10 % (by wt), more preferred at least 15 % (by wt) and most preferred 20 % to 25 % (by wt) of the controlled release matrix formulation.

In such certain embodiments of the invention, the dosage form may comprise, besides the alkyl (ethyl) cellulose and/or the fatty alcohol, fillers and additional substances, such as granulating aids, lubricants, dyes, flowing agents and plasticizers.

Lactose, glucose or saccharose, starches and their hydrolysates, microcrystalline cellulose, cellatose, sugar alcohols such as sorbitol or mannitol, polysoluble calcium salts like calciumhydrogenphosphate, dicalcium- or tricalciumphosphat may be used as fillers.

Povidone may be used as granulating aid.

Highly-dispersed silica (Aerosil^{®}), talcum, corn starch, magnesium oxide and magnesium- or calcium stearate may preferably be used as flowing agents or lubricants. Magnesium stearate and/or calcium stearate can be preferably be used as lubricants. Fats like hydrogenated castor oil can also preferably be used.

According to such certain embodiments, a formulation is especially preferred which comprises ethylcellulose, stearyl alcohol, magnesium stearate as lubricant, lactose as filler and providone as a granulating aid.

In certain such embodiments the present invention the controlled release matrix formulation does not comprise a neutral water insoluble poly (ethyl acrylate methyl acrylate) copolymer and/or a poly(meth)acrylate trimethylammoniummethylacrylate chloride copolymer.

In certain such embodiments of the present invention, the hydrophobic material is an enteric polymer. Examples of suitable enteric polymers include cellulose acetate phthalate, hydroxypropylmethylcellulose phthalate, polyvinylacetate phthalate, methacrylic acid copolymer, shellac, hydroxypropylmethylcellulose succinate, cellulose acetate trimellitate, and mixtures of any of the foregoing.

The dosage form of the present invention can be prepared by extrusion or by granulation in accordance with the teachings of, e.g., U.S. Patent Nos. 5,266,331; 5,958,452; and 5,965,161.

In certain embodiments, in particular embodiments comprising alkyl cellulose, e.g. ethyl cellulose, in combination with fatty alcohols or fatty acids as described above (i.e. the C₁₂ to C₃₆ aliphatic alcohols and the corresponding aliphatic acids), the production of pharmaceutical formulations or preliminary stages thereof, which are in accordance with the invention, by extrusion technology is especially advantageous. In one preferred embodiment, pharmaceutical formulations or preliminary stages thereof are produced by melt extrusion with co- or counter-rotating extruders comprising two screws. Another such preferred embodiment is the production by means of extrusion, with extruders comprising one or more screws. These extruders may also comprise kneading elements.

Extrusion is also a well-established production process in pharmaceutical technology and is well known to the person skilled in the art. The person skilled in the art is well aware that during the extrusion process, various parameters, such as the feeding rate, the screw speed, the heating temperature of the different extruder zones (if available), the water content, etc. may be varied in order to produce products of the desired characteristics.

The aforementioned parameters will depend on the specific type of extruder used. During extrusion the temperature of the heating zones, in which the components of the inventive formulation melt, may be between 40 to 120 °C or between 40 to 160 °C, preferably between 50 to 100 °C or preferably between 50 to 135 °C, more preferably between 50 to 90 °C, even more preferably between 50 to 70 °C and most preferably between 50 to 65 °C, particularly if counter-rotating twin screw extruders (such as a Leistritz Micro 18 GGL) are used. The person skilled in the art is well aware that not every heating zone has to be heated. Particularly behind the feeder where the components are mixed, cooling at around 25 °C may be necessary. The screw speed may vary between 100 to 500 revolutions per minute (rpm), preferably between 100 to 250 rpm, more preferably between 100 to 200 rpm and most preferably around 150 rpm, particularly if counter-rotating twin screw extruders (such as a Leistritz Micro 18 GGL) are used. The geometry and the diameter of the nozzle may be selected as required. The diameter of the nozzle of commonly used extruders typically is between 1 to 10 mm, preferably between 2 to 8 mm and most preferably between 3 to 5 mm. The ratio of length versus diameter of the screw of extruders that may be used for production of inventive preparations is typically around 40 : 1.

Generally, the temperatures of the heating zones have to be selected such that no temperatures develop that may destroy the pharmaceutically active compounds. The feeding rate and screw speed will be selected such that the pharmaceutically active compounds are released from the preparations produced by extrusion in a sustained, independent and invariant manner. If e.g. the feeding rate is increased, the screw speed may have to be increased correspondingly to ensure the same retardation.

The person skilled in the art knows that all the aforementioned parameters depend on the specific production conditions (extruder type, screw geometry, number of components etc.) and may have to be adapted such that the preparations produced by extrusion provide for the required release.

According to such certain embodiments the C₁₂ to C₃₆ aliphatic alcohol or aliphatic acid melts and the ethylcellulose can be dissolved in said C₁₂ to C₃₆ aliphatic alcohol or aliphatic acid during the melt extrusion process.

Opioid agonists salts useful in the present invention include, but are not limited to, pharmaceutically acceptable salts of any of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts, hydrates and solvates thereof, mixtures of any of the foregoing, and the like. In certain embodiments, the amount of the opioid agonist in the dosage form may be about 75 ng to 750 mg.

Opioid antagonist or pharmaceutically acceptable salts thereof useful in combination with opioid agonists or pharmaceutically acceptable salts thereof as described above are naloxone, naltrexone and nalorphine or pharmaceutically acceptable salts thereof. Preferred is the combination of oxycodone HCl and naloxone HCl in an amount ratio of 2:1.

In certain embodiments, the opioid is selected from codeine, morphine, oxycodone, hydrocodone, hydromorphone, or oxymorphone or pharmaceutically acceptable salts thereof.

In certain other embodiments other therapeutically active agents / actives may be used in accordance with the present invention, either in combination of opiods or instead of opioids. Examples of such therapeutically active agents include antihistamines (e.g., dimenhydrinate, diphenhydramine, chlorpheniramine and dexchlorpheniramine maleate), non -steroidal anti-inflammatory agents (e.g., naproxen, diclofenc, indomethacin, ibuprofen, sulindac), anti-emetics (e.g., metoclopramide, methylnaltrexone), anti-epileptics (e.g., phenytoin, meprobmate and nitrazepam), vasodilators (e.g., nifedipine, papaverine, diltiazem and nicardipine), anti-tussive agents and expectorants (e.g. codeine phosphate), anti-asthmatics (e.g. theophylline), antacids, anti-spasmodics (e.g. atropine, scopolamine), antidiabetics (e.g., insulin), diuretics (e.g., ethacrynic acid, bendrofluthiazide), anti-hypotensives (e.g., propranolol, clonidine), antihypertensives (e.g., clonidine, methyldopa), bronchodilatiors (e.g., albuterol), steroids (e.g., hydrocortisone, triamcinolone, prednisone), antibiotics (e.g., tetracycline), antihemorrhoidals, hypnotics, psychotropics, antidiarrheals, mucolytics, sedatives, decongestants, laxatives, vitamins, stimulants (including appetite suppressants such as phenylpropanolamine), as well as pharmaceutically acceptable salts, hydrates, and solvates of the same.

The present invention is also directed to the dosage forms utilizing active agents such as for example, benzodiazepines, barbiturates or amphetamines. These may be combined with the respective antagonists

The term "benzodiazepines" refers to benzodiazepines and drugs that are derivatives of benzodiazepine that are able to depress the central nervous system. Benzodiazepines include, but are not limited to, alprazolam, bromazepam, chlordiazepoxied, clorazepate, diazepam, estazolam, flurazepam, halazepam, ketazolam, lorazepam, nitrazepam, oxazepam, prazepam, quazepam, temazepam, triazolam, methylphenidate as well as pharmaceutically acceptable salts, hydrates, and solvates and mixtures thereof. Benzodiazepine antagonists that can be used in the present invention include, but are not limited to, flumazenil as well as pharmaceutically acceptable salts, hydrates, and solvates.

Barbiturates refer to sedative-hypnotic drugs derived from barbituric acid (2, 4, 6,-trioxohexahydropyrimidine). Barbiturates include, but are not limited to, amobarbital, aprobarbotal, butabarbital, butalbital, methohexital, mephobarbital, metharbital, pentobarbital, phenobarbital, secobarbital and as well as pharmaceutically acceptable salts, hydrates, and solvates mixtures thereof. Barbiturate antagonists that can be used in the present invention include, but are not limited to, amphetamines as well as pharmaceutically acceptable salts, hydrates, and solvates.

Stimulants refer to drugs that stimulate the central nervous system. Stimulants include, but are not limited to, amphetamines, such as amphetamine, dextroamphetamine resin complex, dextroamphetamine, methamphetamine, methylphenidate as well as pharmaceutically acceptable salts, hydrates, and solvates and mixtures thereof. Stimulant antagonists that can be used in the present invention include, but are not limited to, benzodiazepines, as well as pharmaceutically acceptable salts, hydrates, and solvates as described herein.

In certain embodiments, the opioid is hydromorphone hydrochloride in an amount, e.g., of 2 mg, 4 mg, 8 mg, 12 mg, 16 mg, 24 mg, 32 mg, 48 mg or 64 mg hydromorphone hydrochloride.

In certain embodiments, the opioid is oxycodone hydrochloride in an amount, e.g., of 5 mg, 10 mg, 15 mg, 20 mg, 30, mg, 40 mg, 45 mg, 60 mg, or 80 mg, 90 mg, 120 mg or 160 mg oxycodone hydrochloride. In certain embodiments, in particular embodiments comprising alkyl cellulose e.g. ethylcellulose in combination with fatty alcohol oxycodone hydrochloride is combined in the above amounts with naloxone hydrochloride in an amount ratio of 2:1.

The present invention will now be more fully described with reference to the accompanying examples. It should be understood, however, that the following description is illustrative only and should not be taken in any way as a restriction of the invention.

### EXAMPLES OF THE INVENTION

### EXAMPLE 1

### (COMPARATIVE EXAMPLE)

Example 1 is the approved Palladone (sustained release hydromorphone hydrochloride) formulation and contains the following ingredients:

| | |
|---|---|
| Hydromorphone HCl | 12.0mg |
| Eudragit RSPO* | 76.5mg |
| Ethylcellulose | 4.5mg |
| Stearyl alcohol | 27.0mg |

| | |
|---|---|
| * (poly(meth)acrylate with 5% trimethylammoniummethacrylate chloride) | |

The formulation was prepared by the following procedure:
1. Mill the stearyl alcohol.
2. Blend the Hydromorphone HCl, ethylcellulose, Eudragit RSPO and milled stearyl alcohol on a v-blender
3. Extrude the blend from (1) using a ZSE-218 extruder fitted with counter-rotating screws, and a 1mm die plate. Using a pelletizer, cut the strands to create cylindrical pellets approximately 1mm long and 1mm in diameter.

### EXAMPLE 2.1

### EXAMPLE 2.1

The composition of Example 2.1 is summarized below.

| **Ingredient (Trade Name)** | **Amt/unit** **(mg)** | **Amt/batch** **(g)** |
|---|---|---|
| Hydromorphone HCl | 12.0 | 221.1* |
| Ethycellulose (Ethocel Std. Premium 7) | 61.0 | 1,118.3 |
| Glyceryl palmitostearate (Precirol ATO 5) | 27.0 | 495.0 |
| Hydroxypropyl Cellulose (Klucel EF) | 20.0 | 366.7 |
| Total | 120.0 | 2201.1 |

| | | |
|---|---|---|
| * Weigh corrected for water and impurities (99.5% based on Certificate of Analysis) | | |

The processing conditions at the time of sampling are summarized below.
Extruder: Leistritz ZSE 27
Screw Configuration: Counter-rotation

| | | | | | | |
|---|---|---|---|---|---|---|
| Heating Zone | 1 | 2 | 3-6 | 7-8 | 9-10 | 11-12 |
| Temperature (°C) | 15 | 40 | 125 | 125 | 125 | 124-125 |

### Condition #1

Torque (%): 25
Melt Pressure (psi): 480
Feed rate (kg/hour): 2.9
Screw speed (rpm): 90
Die Plate Hole diameter (mm): 1.0 (8-hole die plate)

### Condition #2

Torque (%): 25
Melt Pressure (psi): 520
Feed rate (kg/hour): 4.2
Screw speed (rpm): 90
Die Plate Hole diameter (mm): 1.0 (8-hole die plate)

The processing steps for manufacturing the Hydromorphone HCl 12 mg melt extruded multi particulates are as follows:
1. Screening: The Ethylcellulose, Hydromorphone HCl, Hydroxypropyl Cellulose and Glyceryl Palmitostearate were screened through a #20 US mesh screen (in that order).
2. Blending: The materials screened in Step 1 were loaded into an 8 qt. V-blender with intensifier bar and blended for 10 minutes at ambient temperature.
3. Extrusion: Materials blended in Step 2 were metered into a twin screw extruder fitted with a die and processed into approximately 1 mm strands. The extruder was set on counter-rotation with zone (barrel) temperatures ranged from 15°C to 125°C.
4. Cooling: The strands were cooled on a conveyor at ambient temperature.
5. Pelletizing: The cooled strands were cut into pellets approximately 1 mm in length using a pelletizer.
6. Screening: The pellets were screened through a #16 US mesh screen and a #20 US mesh screen. The pellets retained on the #20 US mesh screen were collected.

### EXAMPLE 2.2

Example 2.2 compares the impact of various concentrations of ethanol in simulated gastric fluid (500 ml in Example 1; 900 ml in Example 2.1) using a USP Apparatus I (basket) apparatus at 100 rpm at 37 degrees C°on the dissolution of the current Palladone formulation and the formulation of Example 2.1 containing the same concentration of hydromorphone (19% w/w). The current Palladone formulation contains an ammonio methacrylate copolymer as the primary release-rate controlling excipient whereas the formulation of Example 2.1 contains ethylcellulose. The results are summarized below.

| Bath | Vessel | 60 minutes |
|---|---|---|
| Example 1 | 1 (SGF Media) | 11% |
| | 2 (10% EtOH in SGF) | 39% |
| | 3 (15% EtOH in SGF) | 64% |
| | 4 (20% EtOH in SGF) | 88% |
| | 5 (40% EtOH in SGF) | 97% |
| Example 2.1 | 1 (SGF Media, pH=1.27) | 13% |
| | 2 (11% EtOH in SGF) | 15% |
| | 3 (20% EtOH in SGF) | 19% |
| | 4 (25% EtOH in SGF) | 23% |
| | 5 (35% EtOH in SGF) | 33% |
| | 6 (SIF Media, pH=7.43) | 13% |

The data show that the formulation of Example 2.1 is more resistant to increases in the drug release in the presence of ethanol. For example, for the current Palladone formulation, concentrations of 20% ethanol in SGF resulted in 8x the amount of hydromorphone to be released in one hour compared to the amount released in SGF. The same concentration of ethanol results in an increase of approximately 1.5x the amount of hydromorphone release for the formulation of Example 2.1 containing ethylcellulose as the rate limiting polymer.

### EXAMPLE 3

### EXAMPLE 3.1

The composition of Example 3.1 is summarized below.

| **Ingredient (Trade Name)** | **Amt/unit** **(mg)** | **Batch** **(gm)** |
|---|---|---|
| Hydromorphone HCl | 12.0 | 168.84* |
| Ethycellulose (Ethocel Std. Premium 7) | 61.0 | 854.0 |
| Hydrogenated Castor Oil | 27.0 | 378.0 |
| Hydroxypropyl Cellulose (Klucel EF) | 20.0 | 280.0 |
| Total | 120.0 | 1680.84 |

| | | |
|---|---|---|
| * weight corrected for water and impurities- 99.5% based on Certificate of Analysis | | |

The processing conditions at the time of sampling are summarized below.
Extruder: Leistritz ZSE 27
Screw Configuration: Counter-rotation

| | | | | | | |
|---|---|---|---|---|---|---|
| Heating Zone | 1 | 2 | 3-6 | 7-8 | 9-10 | 11-12 |
| Temperature (°C) | 15 | 45 | 100-125 | 100-125 | 100-125 | 100-125 |

### Condition #1 (Barrel temp 100C)

Torque (%): 46
Melt Pressure (psi): 2000
Feed rate (kg/hour): 2.9
Screw speed (rpm): 90
Die Plate Hole diameter (mm): 1.0 (8-hole die plate)

### Condition #2 (Barrel temp 125C)

Torque (%): 25
Melt Pressure (psi): 690
Feed rate (kg/hour): 2.9
Screw speed (rpm): 90
Die Plate Hole diameter (mm): 1.0 (8-hole die plate)

The processing steps for manufacturing the Hydromorphone HCl 12 mg melt extruded multi particulates are as follows:
7. Screening: The Ethylcellulose, Hydromorphone HCl, Hydroxypropyl Cellulose and Hydrogenated Castor Oil were screened through a #20 US mesh screen (in that order).
8. Blending: The materials screened in Step 1 were loaded into an 8 qt. V-blender with intensifier bar and blended for 10 minutes at ambient temperature.
9. Extrusion: Materials blended in Step 2 were metered into a twin screw extruder fitted with a die and processed into approximately 1 mm strands. The extruder was set on counter-rotation with zone (barrel) temperatures ranged from 15°C to 125°C.
10. Cooling: The strands were cooled on a conveyor at ambient temperature.
11. Pelletizing: The cooled strands were cut into pellets approximately 1 mm in length using a pelletizer.
12. Screening: The pellets were screened through a #16 US mesh screen and a #20 US mesh screen. The pellets retained on the #20 US mesh screen were collected.

### Testing - Extraction in water at RT and Elevated Temperature

### Procedure

### EXAMPLE 3.2

Example 3.2 compares the resistance to hot water extraction. Place one dosage unit of each drug product into two separate glass scintillation vials and label the vials 1 and 2. Add 10 mL of extraction solvent to each vial. If specified, place the vials in a water bath set to a specified temperature (50, 75 or 100 °C) for 5 minutes. Place both vials on a laboratory wrist-action shaker and remove vial 1 after 15 minutes and vial 2 after 2 hours. Samples at room temperature are placed directly onto the shaker. The experimental set-up is listed below.

| Experiment: | Extraction Solvent | Extraction Temperature | Heating Time | Shaking Times |
|---|---|---|---|---|
| A | Water | Room Temperature | N/A | 15 minutes & 2 hours |
| B | Water | 50 °C | 5 minutes | 15 minutes & 2 hours |
| C | Water | 75 °C | 5 minutes | 15 minutes & 2 hours |
| D | Water | 100 °C | 5 minutes | 15 minutes & 2 hours |

The results of the extraction tests are presented below.

| | | % hydromorphone HCl released (multiple of RT in parentheses) | |
|---|---|---|---|
| Temperature | Time | Example 2.1 | Example 3.1 |
| RT | 15 mins | 6.6 | 11.2 |
| | 120 mins | 6.3 | 12.9 |
| 50C | 15 mins | 4.9 (<1) | 9.0 (<1) |
| | 120 mins | 7.9 (1.3) | 9.6 (<1) |
| 75C | 15 mins | 8.9 (1.3) | 9.5 (<1) |
| | 120 mins | 6.2 (<1) | 11.0 (<1) |
| 100C | 15 mins | 9.1 (1.4) | 12.4 (1.1) |
| | 120 mins | 12.9 (2.0) | 11.8 (<1) |

### EXAMPLE 4

Example 4 is directed to formulations comprising ethylcellulose and poylmethacrylate.

### EXAMPLE 4.1 (Prophetic)

In Example 4.1, the following formulation can be prepared. The formulation may consist of a combination of the following ingredients: drug, ethylcellulose, polymethacrylate, and hydroxypropyl cellulose. An example formulation is presented below.

| **Ingredient** | **g per batch** | **% per batch** | **Ingredient Function** | **Examples** |
|---|---|---|---|---|
| Drug | 120 g | 13.6% | Active Pharmaceutical Ingredient | Opioids |
| Ethylcellulose | 520 g | 59.1% | Hydrophobic film forming agent, control release agent | Ethocel |
| Polymethacrylate (aqueous dispersion) | 200 g¹ (500 g) | 22.7% | Permeable, flexible film forming agent, control release agent | Eudragit NE40D |
| Hydroxypropyl Cellulose | 40 g | 4.6% | Granulating binder | Klucel |

| | | | | |
|---|---|---|---|---|
| ¹ 200 g of solids from an aqueous dispersion containing 40% solids. | | | | |

The range of materials that could be used within this formulation may include other control release agents such as methacrylic acid copolymers (Eudragits), and other cellulose based binding agents such as methylcellulose (Methocel) or hydroxyethyl cellulose (Natrosol)..

The manufacturing process utilizes standard / conventional pharmaceutical processes: , wet granulation, drying, milling, and compression. The granulation process produces a typical granulation (i.e., it resembles a free flowing granular powder); however, when the granulation is compressed, the granules fuse together creating a hard tablet which is resistant to tampering. The manufacturing process is described below.
a. Dry mix the ethylcellulose, API (or spray dried lactose for placebo evaluation) and hydroxypropyl cellulose in a low/high shear mixer.
b. While mixing, add the polymethacrylate (aqueous dispersion) and continue mixing in the low/high shear mixer until a granulation forms.
c. Dry the wet granulation in a fluid bed dryer (or screen on oven trays and dry).
d. Based on the required need, the dried granulation can be:
   - compressed directly using a tablet press.
   - sieved to provide specific particle size fractions for compression or for further blending (e.g., lubricant, additional binder).
   - milled to reduce the particle size (creating a more uniform particle size profile) that could be directly compressed, blended with other ingredients (e.g., lubricant, additional binder), or screened to provide specific particle size fractions for compression or for further blending. Milling can be achieved using a screening mill (such as a rotating impeller or oscillating bar).
e. Compress tablets to target weight on a rotary tablet press.

Further examples 4.2 to 4.8 including ethylcellulose and polymethacrylate.are presented below.

### EXAMPLE 4.2

The composition for EXAMPLE 4.2 is below.

| **Ingredient** | **g per batch** | **% per batch** |
|---|---|---|
| Hydromorphone HCl | 150 g | 10 % |
| Microcrystalline Cellulose, Avicel PH 101 | 150 g | 10 % |
| Ethylcellulose, Ethocel Standard 7 | 600 g | 40 % |
| Polymethacrylate (aqueous dispersion) Eudragit NE40D | 450 g¹ (1125 g) | 30 % |
| Hydroxypropyl Cellulose Klucel EF | 150 g | 10 % |

| | | |
|---|---|---|
| ¹ 450 g of solids from an aqueous dispersion containing 40% solids. | | |

The processing steps for manufacturing the Hydromorphone HCl tablets are as follows:
f. Dry mix the Ethylcellulose, Hydromorphone HCl, Microcrystalline Cellulose and Hydroxypropyl Cellulose in a low/high shear mixer.
g. While mixing, add the Polymethacrylate (aqueous dispersion) and continue mixing in the low/high shear mixer until a granulation forms.
h. Dry the wet granulation in a fluid bed dryer (or screen onto oven trays and dry).
i. Mill the dried granulation using a screening mill (such as a rotating impeller or oscillating bar).
j. Compress the milled granulation to target weight on a rotary tablet press.

Tablets were compressed to a weight of 120 mg to target a 12 mg dose. The resultant tablet composition is provided below.

| | **mg / unit** |
|---|---|
| Hydromorphone HCl | 12 |
| MCC (Avicel 101) | 12 |
| Ethocel Standard 7 | 48 |
| Eudragit NE40D | 36 |
| HPC (Klucel EF) | 12 |
| | **120 mg** |

The tablets were tested in vitro using USP Apparatus 2 (paddle) in simulated gastric fluid, simulated intestinal fluid, in 11% Ethanol and 35% Ethanol. The results are presented below.

| | **SGF** | **SIF** | **11% EtOH** | **35% EtOH** |
|---|---|---|---|---|
| 1 hr | 43 | 40 | 42 | 35 |
| 2 hr | 60 | 56 | 59 | 47 |

### EXAMPLES 4.3 to 4.5

Compositions of Examples 4.3, 4.4 and 4.5 are summarized below.

| **EXAMPLE** | **4.3** | | **4.4** | | **4.5** | |
|---|---|---|---|---|---|---|
| **Ingredient** | **g per batch** | **% per batch** | **g per batch** | **% per batch** | **g per batch** | **% per batch** |
| Hydromorphone HCl | 100 g | 11.8% | 100 g | 11.8% | 100 g | 11.8% |
| Ethylcellulose | 600 g | 70.6 % | 500 g | 58.8% | 400 g | 47.1% |
| Polymethacrylate (aqueous dispersion) | 100 g¹ (250 g) | 11.8% | 200 g¹ (500 g) | 23.5% | 300 g¹ (750 g) | 35.3% |
| Hydroxypropyl Cellulose | 50 g | 5.9% | 50 g | 5.9% | 50 g | 5.9% |

| | | | | | | |
|---|---|---|---|---|---|---|
| ¹ Amount of solids from an aqueous dispersion containing 40% solids. | | | | | | |

The processing steps for manufacturing the Hydromorphone HCl tablets are as follows:
a. Dry mix the Ethylcellulose, Hydromorphone HCl, and Hydroxypropyl Cellulose in a low/high shear mixer.
b. While mixing, add the Polymethacrylate (aqueous dispersion) and continue mixing in the low/high shear mixer until a granulation forms.
c. Dry the wet granulation in a fluid bed dryer (or screen onto oven trays and dry).
d. Mill the dried granulation using a screening mill (such as a rotating impeller or oscillating bar).
e. Compress the milled granulation to target weight on a single station tablet press.
f. Cure the tablets in an oven.

Tablets were compressed to a weight of 102 mg to target a 12 mg dose as presented below.

| **EXAMPLE** | **4.3** | | **4.4** | | **4.5** | |
|---|---|---|---|---|---|---|
| **Ingredient** | **Granulation mg / unit** | **Tablet mg / unit** | **Granulation mg / unit** | **Tablet mg / unit** | **Granulation mg / unit** | **Tablet mg / unit** |
| Hydromorphone HCl | 10 | 12 | 10 | 12 | 10 | 12 |
| Ethylcellulose | 60 | 72 | 50 | 60 | 40 | 48 |
| Polymethacrylate (aqueous dispersion) | 10 | 12 | 20 | 24 | 30 | 36 |
| Hydroxypropyl Cellulose | 5 | 6 | 5 | 6 | 5 | 6 |
| Total | 85 | 102 | 85 | 102 | 85 | 102 |

The tablets were tested in vitro using a USP Apparatus 2 (paddle) at 50 rpm at 37 degrees C in 900 ml of simulated gastric fluid to evaluate drug release. Drug release data was collected using a UV spectrometer flow through system at a wavelength of 280 nm. The results are presented below.

| Time (hours) | **4.3** | **4.4** | **4.5** |
|---|---|---|---|
| 0.5 | 15% | 17% | 16% |
| 1 | 20% | 24% | 23% |
| 1.5 | 24% | 29% | 28% |
| 2 | 28% | 34% | 33% |
| 2.5 | 31% | 39% | 37% |

Compositions of Examples 4.6, 4.7 and 4.8 are summarized below.

| **EXAMPLE** | **4.6** | | **4.7** | | **4.8** | |
|---|---|---|---|---|---|---|
| **Ingredient** | **g per batch** | **% per batch** | **g per batch** | **% per batch** | **g per batch** | **% per batch** |
| Oxycodone HCl | 100 g | 10 % | 100 g | 10 % | 100 g | 10 % |
| Ethylcellulose | 550 g | 55% | 350 g | 35% | 150 g | 15% |
| Polymethacrylate (aqueous dispersion) | 250 g¹ (625 g) | 25% | 250 g¹ (625 g) | 25% | 250 g¹ (625 g) | 25% |
| Hydroxypropyl Cellulose | 100 g | 10 % | 300 g | 30 % | 500 g | 50 % |

| | | | | | | |
|---|---|---|---|---|---|---|
| ¹ Amount of solids from an aqueous dispersion containing 40% solids. | | | | | | |

The processing steps for manufacturing the Oxycodone HCl tablets are as follows:
a. Dry mix the Ethylcellulose, Oxycodone HCl, and Hydroxypropyl Cellulose in a low/high shear mixer.
b. While mixing, add the Polymethacrylate (aqueous dispersion) and continue mixing in the low/high shear mixer until a granulation forms.
c. Dry the wet granulation in a fluid bed dryer (or screen onto oven trays and dry).
d. Mill the dried granulation using a screening mill (such as a rotating impeller or oscillating bar).
e. Compress the milled granulation to target weight on a single station tablet press.

Tablets were compressed to a weight of 100 mg to target a 10 mg dose as presented below.

| **Example** | **4.6** | **4.7** | **4.8** |
|---|---|---|---|
| **Ingredient** | **Tablet mg / unit** | **Tablet mg *l* unit** | **Tablet mg / unit** |
| Hydromorphone HCl | 10 | 10 | 10 |
| Ethylcellulose | 55 | 35 | 15 |
| Polymethacrylate (aqueous dispersion) | 25 | 25 | 25 |
| Hydroxypropyl Cellulose | 10 | 30 | 50 |
| Total | 100 | 100 | 100 |

The tablets were tested in vitro using a USP Apparatus 2 (paddle) at 50 rpm at 37 degree C in 900 ml of simulated gastric fluid to evaluate drug release. Drug release data was collected using a UV spectrometer flow through system at a wavelength of 230 nm. In addition, Example 4.8 was tested in 40% Ethanol / SGF to evaluate the impact of ethanol on drug release. The results are presented below.

| Time (hours) | **4.6** | **4.7** | **4.8** | **4.8 in 40% Ethanol / SGF** |
|---|---|---|---|---|
| 1 | 43% | 55% | 36% | 45% |
| 2 | 71% | 77% | 50% | 66% |
| 4 | 94% | 92% | 67% | n/a |
| 6 | 95% | 97% | 79% | n/a |
| 8 | 95% | 98% | 87% | n/a |
| 12 | 95% | 99% | 96% | n/a |

### EXAMPLE 5

Compositions A through F of Example 5 are summarized below.

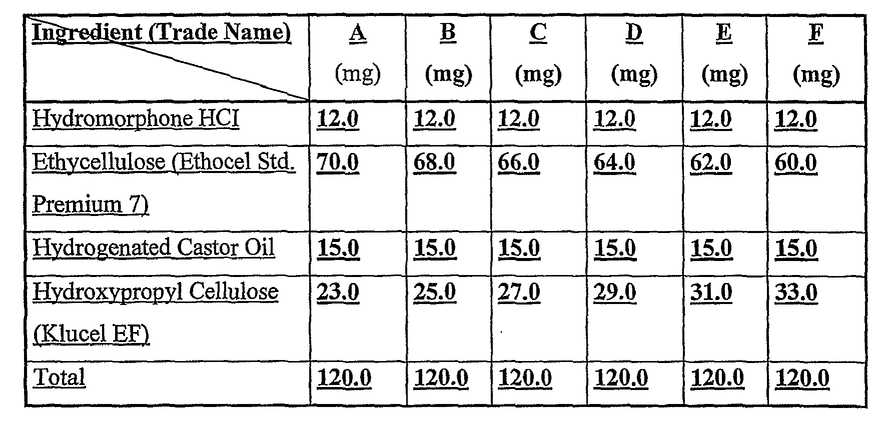

The processing conditions at the time of sampling are summarized below:
Extruder: Leistritz ZSE 27
Screw Configuration: Counter-rotation

| | | | | | | |
|---|---|---|---|---|---|---|
| Heating Zone | 1 | 2 | 3-6 | 7-8 | 9-10 | 11-12 |
| Temperature (°C) | 15 | 40 | 125 | 125 | 125 | 135 |

### Conditions.

Feed rate (kg/hour): 4.2
Screw speed (rpm): 90
Die Plate Hole diameter (mm): 1.0 (8-hole die plate)

The processing steps for manufacturing the Hydromorphorphone HCl 12 mg melt extruded multi particulates are as follows:
1. Screening: The Ethylcellulose, Hydromorphone HCl and Hydroxypropylcellulose were screened though a #20 US mesh screen.
2. Blending: The materials screened in Step 1 were loaded into an 8 qt. V-blender with intensifier bar and blended for 10 minutes at ambient temeperature.
3. Extrusion: Materials blended in Step 2 were metered into a twin screw extruder fitted with a die and processed in to approximately 1 mm strands. The extruder was set on counter-rotation with zone (barrel) set-temperatures ranged from 15°C to 135°C.
4. Cooling: The strands were cooled on a conveyor at ambient temperature.
5. Pelletizing: The cooled strands were cut into pellets approximately 1 mm in length using a pelletizer.
6. Screening: The pellets were screened through a # 16 US mesh screen and a #20 US mesh screen. The pellets retained on the #20 US mesh screen were collected.

Formulations A, C and F were tested in vitro using a USP Apparatus I (basket) apparatus at 100 rpm at 37 degrees °C in various concentrations of ethanol in 500 ml simulated gastric fluid in order to determine the impact of ethanol on drug release. The results are presented below.

| | **% Hydromorphone Released in 60 minutes (ratio to amount released in SGF)** | | | |
|---|---|---|---|---|
| Ethanol concentration (%) | **A** | **C** | **F** | **Example 1** |
| 0 | 15 | 14 | 20 | 11 |
| 5 | 14 | 16 | 20 | 18* (1.6) |
| 10 | 14 | 15 | 22 | 39 (3.5) |
| 20 | 14 (0.9) | 17 (1.2) | 20 (1) | 88 (8.0) |
| 30 | 27 (1.8) | 30 (2.1) | 34 (1.7) | - |
| 40 | 39 (2.6) | 45 (3.2) | 55 (2.8) | 97 (8.8) |

The results above show that hydromorphone release from formulations A, C, and F was unchanged in ethanol concentrations up to and including 20% v/v. This is an improvement on Example 1 (the current Palladone formulation) which has an eight-fold increase in hydromorphone release under these conditions. At 30% and 40% v/v ethanol there was an increase in release for formulations A, C and F but the increases relative to release in SGF were lower than those obtained with Example 1 (the current Palladone formulation).

### EXAMPLE 6

Oxycodone/naloxone dosage form comprising 10 mg oxycodone hydrochloride and 5 mg naloxone hydrochloride

| Component | weight [mg/tablet] |
|---|---|
| Oxycodone hydrochloride¹⁾ | 10.50 |
| corresponding to | |
| Oxycodone hydrochloride anhydrous | 10.00 |
| naloxone hydrochloride dihydrate | 5.45 |
| corresponding to | |
| Naloxone hydrochloride anhydrous | 5.00 |
| Povidone K30 | 5.00 |
| Ethyl cellulose 45 cp | 10.00 |
| Stearyl alcohol | 25.00 |
| Lactose monohydrate | 64.25 |
| Talc | 2.50 |
| Magnesium-Stearate | 1.25 |
| film coating opadry II HP white - 85F18422° | 3.72 |

| | |
|---|---|
| 1) calculated based on expected moisture content ° qualitative composition: see below | |

### EXAMPLE 7

Oxycodone/naloxone dosage form comprising 20 mg oxycodone hydrochloride and 10 mg naloxone hydrochloride

| Component | weight [mg/tablet] |
|---|---|
| Oxycodone hydrochloride¹⁾ | 21.00 |
| corresponding to | |
| Oxycodone hydrochloride anhydrous | 20.00 |
| naloxone hydrochloride | 10.90 |
| corresponding to | |
| Naloxone hydrochloride anhydrous | 10.00 |
| Povidone K30 | 7.25 |
| Ethyl cellulose 45 cp | 12.00 |
| Stearyl alcohol | 29.50 |
| Lactose monohydrate | 54.50 |
| Talc | 2.50 |
| Magnesium-Stearate | 1.25 |
| film coating opadry II HP pink 85F24151° | 4.17 |

| | |
|---|---|
| 2) calculated based on expected moisture content ° qualitative composition: see below | |

### EXAMPLE 8

Oxycodone/naloxone dosage form comprising 40 mg oxycodone hydrochloride and 20 mg naloxone hydrochloride

| Component | weight [mg/tablet] |
|---|---|
| Oxycodone hydrochloride¹⁾ | 42.00 |
| corresponding to | |
| Oxycodone hydrochloride anhydrous | 40.00 |
| naloxone hydrochloride dihydrate | 21.80 |
| corresponding to | |
| Naloxone hydrochloride anhydrous | 20.00 |
| Povidone K30 | 14.50 |
| Ethyl cellulose 45 cp | 24.00 |
| Stearyl alcohol | 59.00 |
| Lactose monohydrate | 109.00 |
| Talc | 5.00 |
| Magnesium-Stearate | 2.5 |
| film coating opadry II HP yellow 85F32109° | 8.33 |
| | |

| | |
|---|---|
| 3) calculated based on expected moisture content ° qualitative composition: see below | |

### Qualitative composition of the film coat

| **Opadry II HP** | **white 85F18422** | **pink 85F24151** | **yellow 85F32109** | **Reference to Standard** |
|---|---|---|---|---|
| Polyvinylalcohol part. hydrolized | + | + | + | Ph. Eur. * |
| Titanium dioxide (E 171) | + | + | + | Ph. Eur. * |
| Macrogol 3350 | + | + | + | Ph. Eur. * |
| Talcum | + | + | + | Ph. Eur. * |
| Iron oxide red (E 172) | | + | | NF* /EC Directive |
| Iron oxide yellow (E 172) | | | + | NF* /EC Directive |
| | | | | * current Edition |

The above described dosage forms were prepared by melt extrusion.
Oxycodone hydrochloride and naloxone hydrochloride are blended with povidone, ethylcellulose, stearyl alcohol and lactose, the blend is screened to remove agglomerates and further blended. The blend is melt extruded utilizing a heated twin screw extruder, to form strands which are milled to produce granules. The granules are blended with talc and magnesium stearate, compressed into capsule shaped tablets, which are then film coated.

### EXAMPLE 9

### Dissolution test

The dissolution apparatus was assembled in accordance with the USP basket/100rpm/900ml dissolution media method as described e.g. in USP 23. The specified dissolution media were transferred into each vessel with the bath temperature set to 37.0 ± 0.5 °C. All ethanolic media were prepared by transferring the appropriate amount of ethanol in USP Simulated Gastric Fluid (SGF) without pepsin (i.e. 9 mL of ethanol with 891 mL of SGF for a 1% ethanol media).

A single tablet was transferred into each vessel. A sample was drawn from each vessel at four time points: 10, 30, 60 and 120 minutes. Using the HPLC test method samples and (corresponding) standards were injected onto the column to determine the amount of oxycodone HCl and naloxone HCl dissolved.

Twelve different concentrations of ethanol were tested, 0%, 2%, 4%, 8%, 12%, 16%, 20%, 24%, 28%, 32%, 36% and 40%.

### Example 9 / dissolution results of Example 6

| **dissolution (%) naloxone HCl (ratio to amount released in SGF 0 % alcohol)** | | | | | **dissolution (%) oxycodone HCl (ratio to amount released in SGF 0 % alcohol)** | | | |
|---|---|---|---|---|---|---|---|---|
| % ethanol | 10 min. | 30 min. | 60 min. | 120 min. | 10min. | 30 min. | 60 min. | 120 min. |
| 0 | 17 | 29 | 40 | 53 | 16 | 28 | 39 | 53 |
| 2 | 17 | 29 | 39 | 53 | 15 | 28 | 38 | 52 |
| 4 | 18 | 29 | 39 | 52 | 15 | 27 | 37 | 51 |
| 8 | 16 | 27 | 37 | 50 | 14 | 26 | 36 | 49 |
| 12 | 16 | 27 | 36 | 52 | 13 | 25 | 34 | 47 |
| 16 | 14 | 27 | 38 | 50 | 12 | 24 | 33 | 45 |
| 20 | 15 | 25 | 34(0.9) | 45 | 12 | 23 | 32(0.8) | 44 |
| 24 | 13 | 25 | 34 | 45 | 10 | 23 | 32 | 44 |
| 28 | 14 | 25 | 34 | 45 | 11 | 22 | 32 | 44 |
| 32 | 13 | 24 | 33 | 44 | 10 | 22 | 32 | 44 |
| 36 | 13 | 24 | 33 | 45 | 11 | 22 | 31 | 44 |
| 40 | 13 | 24 | 33(0.8) | 45 | 10 | 22 | 31(0.8) | 44 |

### Example 9 / dissolution results of Example 7

| **dissolution (%) naloxone HCl(ratio to amount released in SGF)** | | | | | **dissolution (%) oxycodone HCl(ratio to amount released in SGF)** | | | |
|---|---|---|---|---|---|---|---|---|
| % ethanol | 10 min. | 30 min. | 60 min. | 120 min. | 10 min. | 30 min. | 60 min. | 120 min. |
| 0 | 16 | 28 | 39 | 52 | 16 | 28 | 39 | 53 |
| 2 | 16 | 28 | 39 | 52 | 15 | 28 | 38 | 52 |
| 4 | 16 | 28 | 38 | 52 | 15 | 28 | 38 | 52 |
| 8 | 14 | 26 | 36 | 51 | 14 | 25 | 36 | 49 |
| 12 | 16 | 26 | 37 | 49 | 13 | 25 | 34 | 47 |
| 16 | 15 | 26 | 36 | 49 | 13 | 24 | 34 | 47 |
| 20 | 15 | 24 | 33(0,8) | 45 | 13 | 23 | 32(0,8) | 43 |
| 24 | 14 | 23 | 33 | 45 | 12 | 22 | 32 | 44 |
| 28 | 14 | 24 | 33 | 45 | 13 | 23 | 32 | 44 |
| 32 | 13 | 24 | 32 | 44 | 12 | 22 | 31 | 43 |
| 36 | 14 | 24 | 33 | 44 | 12 | 22 | 32 | 44 |
| 40 | 14 | 24 | 34(0.9) | 46 | 12 | 23 | 33(0.9) | 45 |

### Example 9 / dissolution results of Example 8

| **dissolution (%) naloxone HCl(ratio to amount released in SGF)** | | | | | **dissolution (%) oxycodone HCl (ratio to amount released in SGF)** | | | |
|---|---|---|---|---|---|---|---|---|
| % ethanol | 10 min. | 30 min. | 60 min. | 120 min. | 10 min. | 30 min. | 60 min. | 120 min. |
| 0 | 13 | 23 | 34 | 45 | 13 | 24 | 33 | 46 |
| 2 | 13 | 23 | 33 | 46 | 13 | 23 | 33 | 46 |
| 4 | 13 | 23 | 32 | 44 | 12 | 23 | 32 | 44 |
| 8 | 13 | 23 | 32 | 43 | 12 | 22 | 31 | 43 |
| 12 | 12 | 22 | 30 | 42 | 11 | 21 | 29 | 40 |
| 16 | 11 | 21 | 30 | 41 | 10 | 20 | 28 | 40 |
| 20 | 11 | 20 | 28(0.8) | 39 | 11 | 19 | 27(0.8) | 38 |
| 24 | 11 | 20 | 28 | 38 | 11 | 19 | 27 | 38 |
| 28 | 12 | 20 | 27 | 38 | 11 | 19 | 27 | 38 |
| 32 | 11 | 20 | 28 | 38 | 10 | 19 | 27 | 37 |
| 36 | 11 | 19 | 28 | 38 | 11 | 19 | 27 | 38 |
| 40 | 11 | 19 | 27(0.8) | 38 | 10 | 19 | 27(0.8) | 37 |

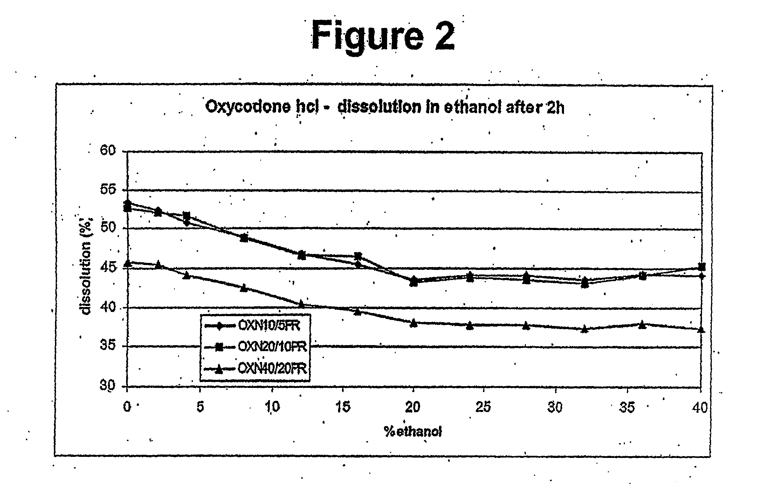

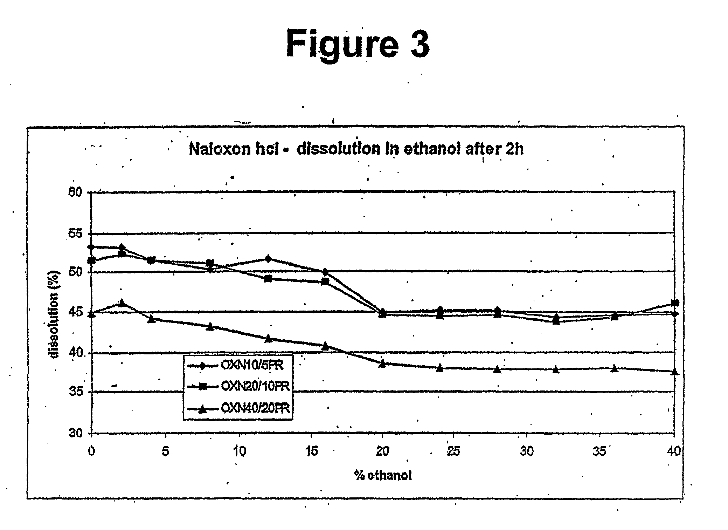

The dissolution results of Example 6 to 8 are shown in Fig. 2 and 3. Fig. 2 shows the dissolution (%) of oxycodone after two hours for example 6 (OX/N 10/5 PR), example 7 (OX/N 20/10 PR) and example 9 (OX/N 20/40 PR). Fig. 3 shows the corresponding dissolution (%) of naloxone after two hours.

Between 0 and 20 % ethanol, the amount of released active even decreases, while between 20 and 40 % ethanol the release is stable. This can be observed for oxycodone hydrochloride and naloxone hydrochloride with respect to all three dosage forms of examples 6 to 8.

### EXAMPLES 10 TO 13

The compositions of Examples 10 to 13 are below.

| Material | | Examples (% w /w) | | |
|---|---|---|---|---|
| | Example 10 | Example 11 | Example 12 | Example 13 |
| | | | | |
| Oxycodone HCl | 10.0 | 10.0 | 10.0 | 10.0 |
| Ethyl cellulose N10 | 41.8 | nil | 32.0 | nil |
| Eudragit RS PO* | nil | 41.8 | nil | 22.0 |
| Eudragit RL PO* | nil | nil | 10.0 | 20.0 |
| Stearyl alcohol | 14.0 | 14.0 | 14.0 | 14.0 |
| Eudragit NE 40 D* | 34.2 (S), [85.5 (D)] | 34.2 (S), [85.5 (D)] | 34.0 (S), [85.0 (D)] | 34.0 (S), [85.0 (D)] |
| Total | 100 | 100 | 100 | 100 |

| | | | | |
|---|---|---|---|---|
| S = solid weight D = dispersion weight * Eudragit RS PO: poly(meth) acrylate with 5% trimethylammoniummethacrylate chloride * Eudragit RL PO: poly(meth) acrylate with 10% trimethylammoniummethacrylate chloride * Eudragit NE 40 40% dispersion (% w /w), water lost by evaporation neutral poly(ethylacrylate methyl methacrylate) copolymer | | | | |

A procedure for preparing multi particulates of Examples 10 to 13 in the form of pellets is approximately:
Step 1. The oxycodone was blended for 5 minutes with ethyl cellulose and/or Eudragit RS PO/ RL PO and stearyl alcohol in the Oral 10 high shear mixer
Step 2. Eudragit NE 40 D dispersion was slowly added by aid of a peristaltic pump onto the blended materials from Step 1 in the Gral 10 mixing bowl, pre-warmed for Examples 12 and 13 to 29°C, whilst maintaining mixing/chopping.
Step 3. The application of Eudragit NE 40 D was continued until granule formation occurred - all the Eudragit NE 40 D was added.
Step 4. The application of Eudragit NE 40 D was periodically halted to permit scraping of the sides of the mixing bowl.
Step 5. After all the Eudragit NE 40 D had been added, the wet granules were extruded through a conventional extruder and then dried in a fluid bed dryer at approximately 44°C.
Step 6. The dried granules were cooled to room temperature and collected.
Step 7. The granules were then fed at a controlled rate to a Leistritz Micro 18 extruder equipped with a 1.0 mm die-plate, a conveyor and pelletiser and heated stations (zones) torque and melt pressure as follows;.

| **Example** | **Temperature (°C)** | | **Melt Pressure (bar)** | **Torque (%)** |
|---|---|---|---|---|
| | Zones 3-8 | Zones 9-10 | | |
| **10** | 115-120 | 115-120 | 63-72 | 59-62 |
| **11** | 110-115 | 110-115 | 70-72 | 50-60 |
| **12** | 80-105 | 90-100 | 73-86 | 64-72 |
| **13** | 90-100 | 100-110 | 76-96 | 67-85 |

The feed rate was 2.0 to 2.6 kg/hr and the screw speed 100 to 141 rpm. The extruded strands were carried away from the die-head on a conveyer and cut into cylindrical multi particulates.

Hypothetically, an alternate cutting procedure can be considered. Extrudate emerges from the orifices of the die-head of a Leistritz extruder. A rotary cutter with two blades would be used to cut the extruded mix as it emerges under pressure and still molten from the orifices of the die plate. The blades would sweep over the surface of the die-head to pass the orifices. As they expand and cool, the cut extrudate particles would tend to form rounded surfaces.

Hypothetically, although in the above Examples a Leistritz Micro 18 extruder could be used, a larger extruder, for example a Leistritz Micro 27, may be preferred to handle materials requiring a higher torque for processing.

### EXAMPLE 14;

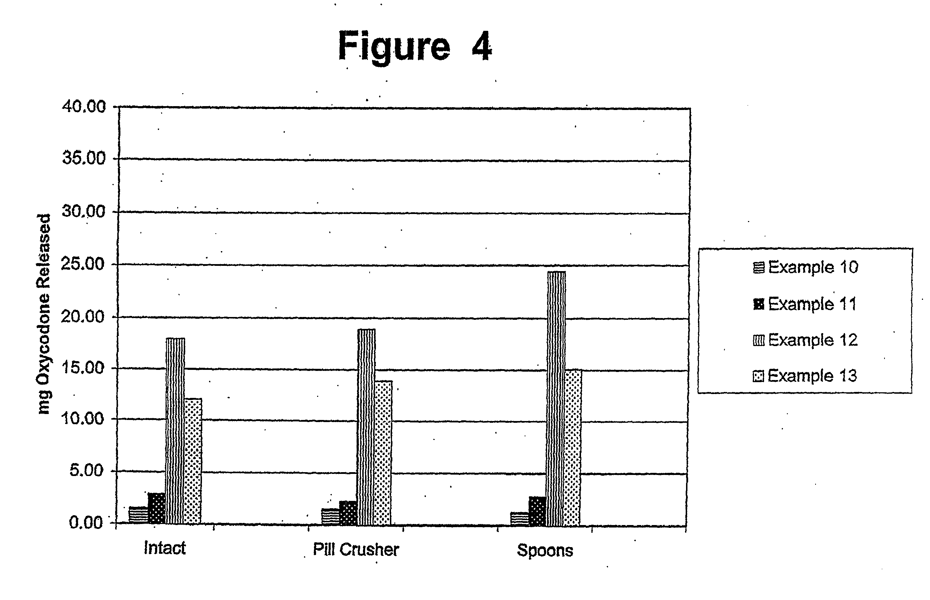

The multi particulates from Examples 10 to 13 were tested to determine their potential for tamper resistance as follows:
1) 400 mg of the multi particulates from Examples 10 to 13 were either crushed between two spoons or in a pill crusher, such as a Pill Pulverizer as sold by Apex Healthcare Products, and then extracted in 2 ml water heated to boiling on a spoon and filtered off. The amounts of oxycodone extracted were then determined by HPLC and detection by UV at 210 nm wavelength and are shown in the chart of Figure 4 and below.

| | **Mean Oxycodone Released (mg)*** | | | |
|---|---|---|---|---|
| | Example 10 | Example 11 | Example 12 | Example 13 |
| Intact | 1.54 | 2.91 | 17.97 | 12.04 |
| Pill Crusher | 1.54 | 2.31 | 18.90 | 13.91 |
| Spoons | 1.25 | 2.75 | 24.42 | 15.07 |

| | | | | |
|---|---|---|---|---|
| * Values are the mean of two repetitions of the test. | | | | |

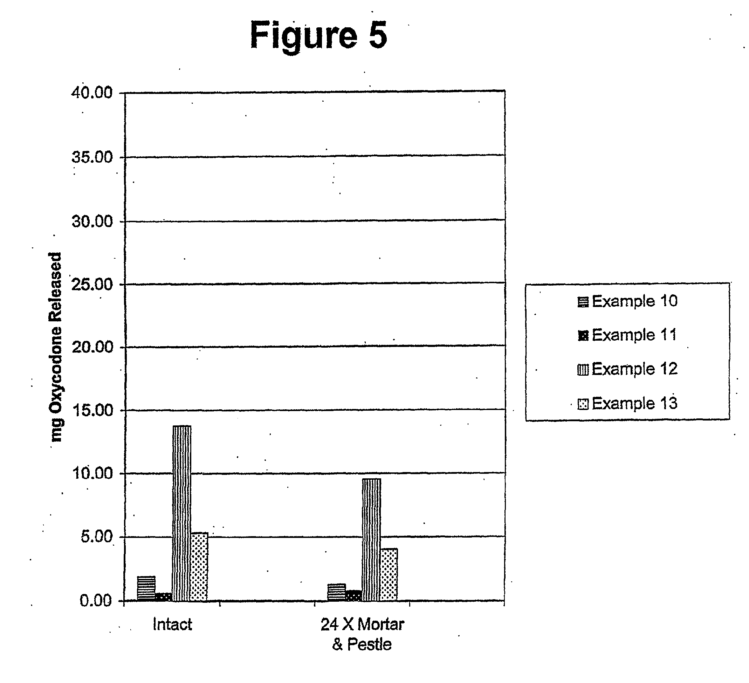

2) 400 mg of the multi particulates from Examples 10 to 13 were subjected to grinding in a mortar and pestle with 24 rotations of the pestle and the product placed in 900 ml water at 37 °C for 45 minutes. The amount of oxycodone dissolved was then determined.by the method described in 1) above and the results are represented in the bar chart of Figure 5 and below.

| | **Mean Oxycodone Released (mg)*** | | | |
|---|---|---|---|---|
| | Example 10 | Example 11 | Example 12 | Example 13 |
| Intact | 1.92 | 0.62 | 13.74 | 5.30 |
| 24x Mortar & Pestle | 1.32 | 0.81 | 9.53 | 4.02 |

| | | | | |
|---|---|---|---|---|
| * Values are the mean of two repetitions of the test. | | | | |

3) In each of extractions a) to e) 400 mg of the multi particulates from one of Examples 10 to 13 were treated respectively as follows: the multi particulates were placed in the solvent indicated in a glass flask which was then heated (if heating is indicated) over a water bath. The flask was then subjected to shaking for the time indicated using a Stuart Scientific Flask Shaker Model SF 1 set at 500 to 600 oscillations per minute. After extraction the amount of oxycodone dissolved was then determined by the method used in 1).
a) 15 minutes shaking in 10 ml water at room temperature;
b) heating for 5 minutes in 10 ml water at 50 °C followed by 15 minutes shaking;
c) heating for 5 minutes in 10 ml water at 75 °C followed by 15 minutes shaking.
d) heating for 5 minutes in 10 ml water at 100 °C followed by 15 minutes shaking.
e) 15 minutes shaking in 10 ml 40 % ethanol at room temperature.

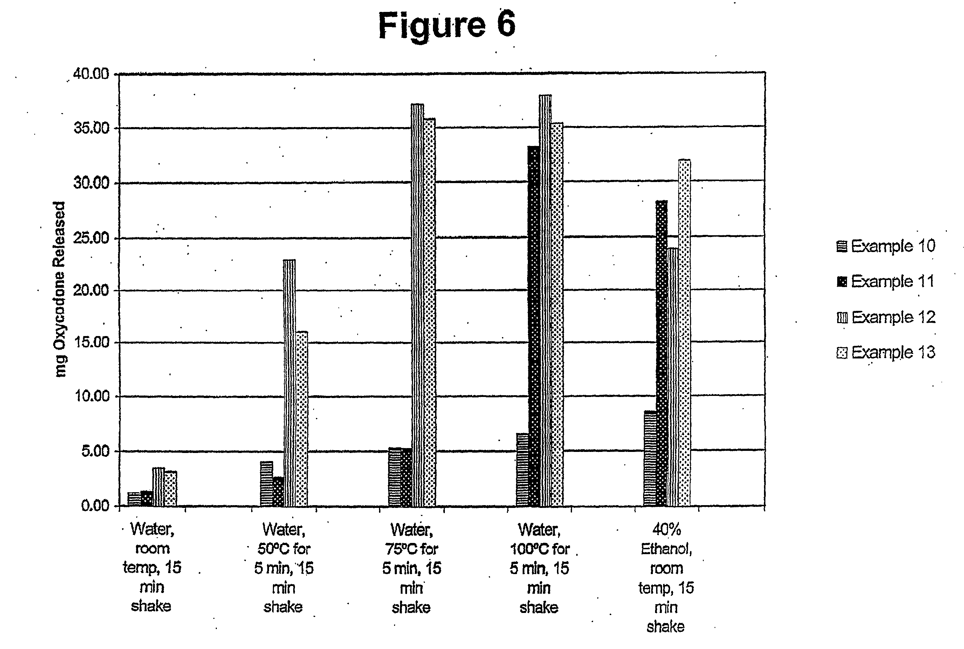

The test results are shown in the attached bar chart of Figure 6 and below.

| | Example 10 | Example 11 | Example 12 | Example 13 |
|---|---|---|---|---|
| 40% ethanol room temp. 15 min shake | 8.54 mg | 28.26 mg | 23.91 mg | 31.85 mg |
| | (21.4% by wt) | (71.7% by wt) | (59.8% by wt) | (79.6% by wt) |
| Water room temp. 15 min shake | 1.22 mg | 1.39 mg | 3.47 mg | 3.11 mg |
| | (3.0% by wt) | (3.5% by wt) | (8.7% by wt) | (7.8% by wt) |
| Water 50°C for 5 min 15 min shake | 4.08 | 2.71 | 22.94 | 16.02 |
| | (10.2 % by wt) | (6.8 % by wt) | (57.4 %by wt) | (40.1 % by wt) |
| Water 75 °C for 5 min 15 min shake | 5.34 | 5.28 | 37.09 | 35.78 |
| | (13.3 % by wt) | (13.2 % by wt) | (72.3 % by wt) | (89.5 % by wt) |
| Water 100 °C for 5 min 15 min shake | 6.60 | 33.18 | 37.92 | 35.36 |
| | (16.5 % by wt) | (82.9 % by wt) | (94.8 % by wt) | (88.4 % by wt) |

### EXAMPLES 15 TO 24

Further Examples 15 to 24 are presented in Table 1 below.

### EXAMPLE 25

### Alcohol Extraction Test and dissolution profiles

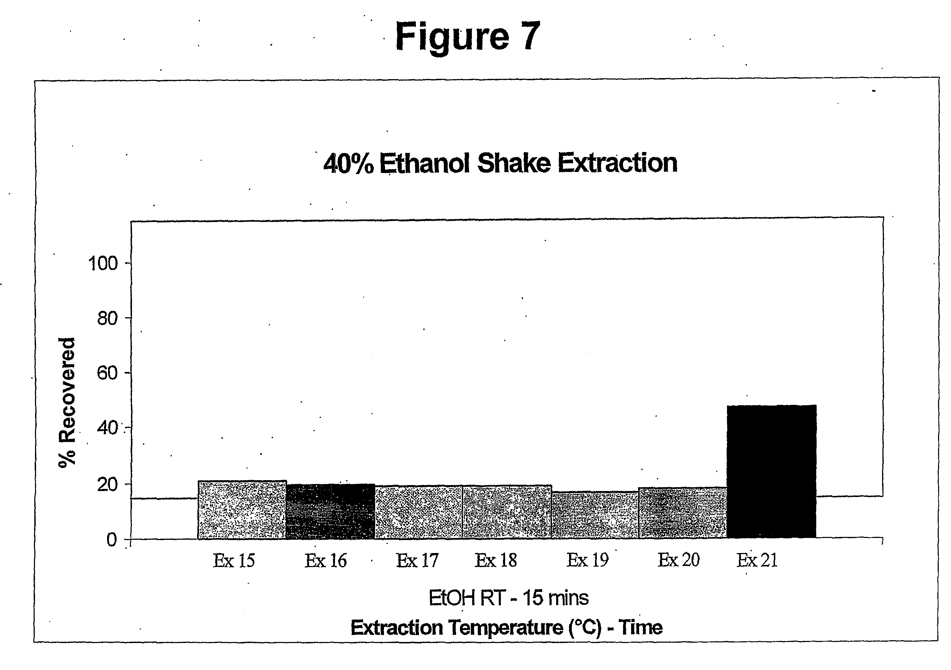

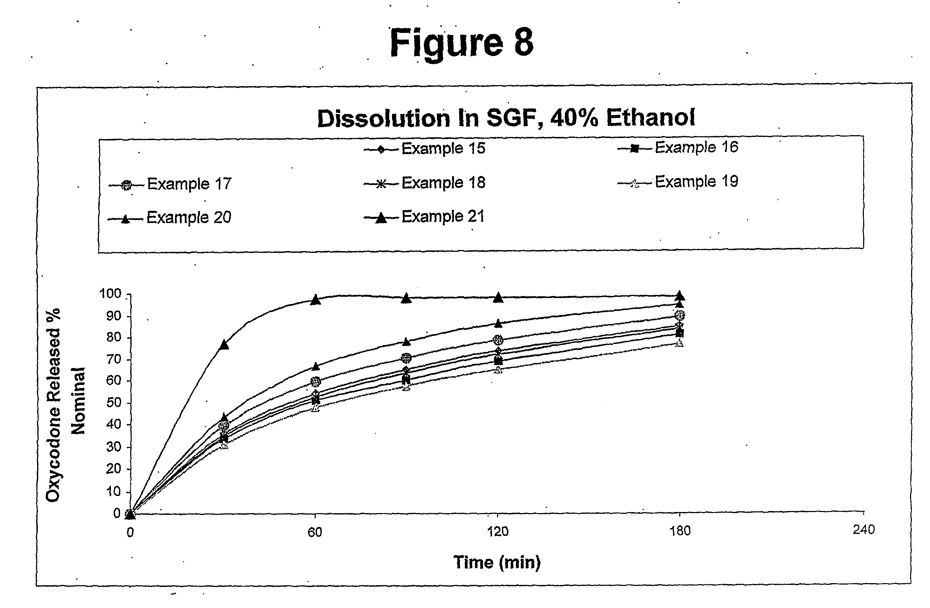

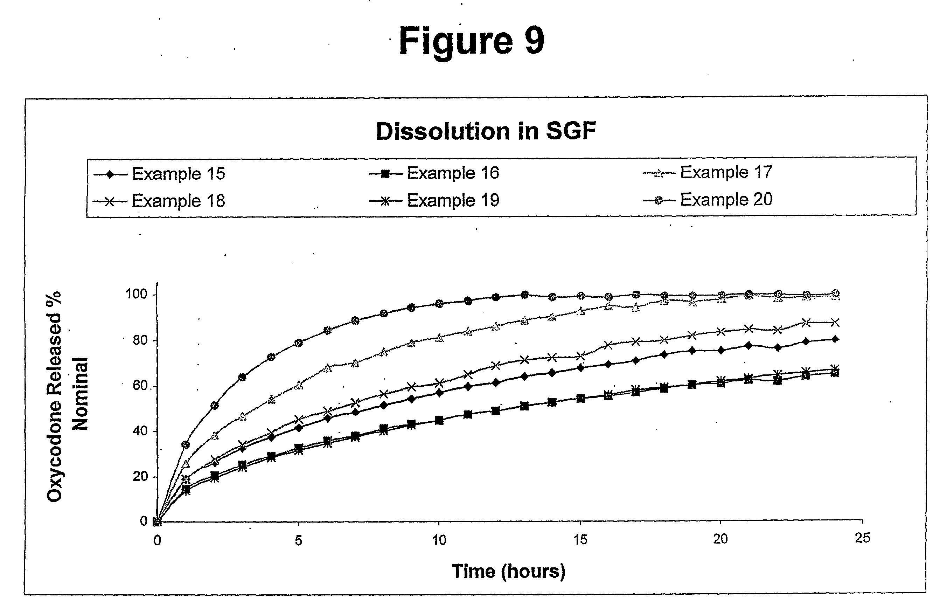

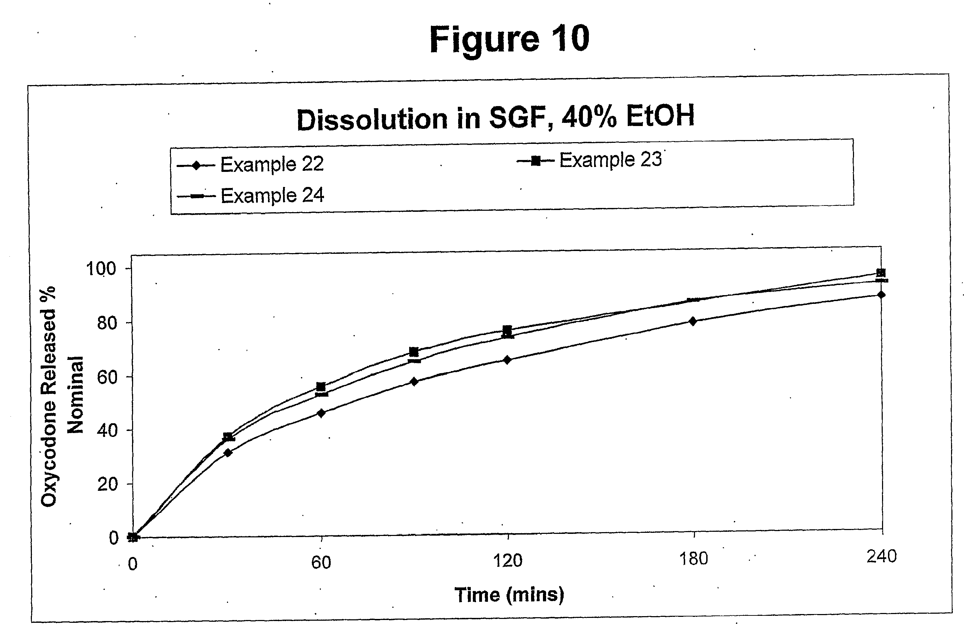

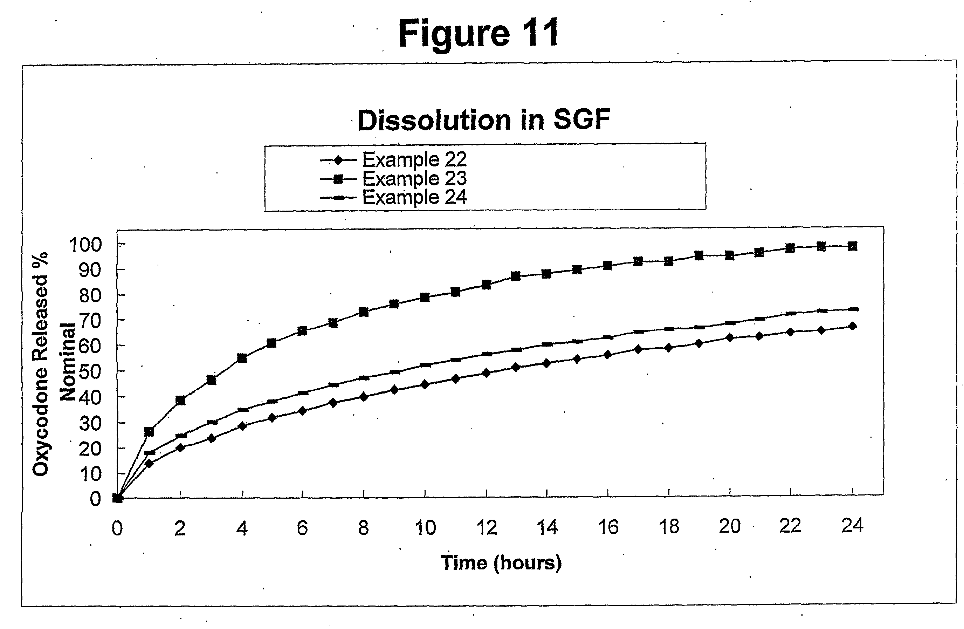

400 mg of the multi particulates from one of Examples 15 to 21 were placed in 10 ml 40% ethanol at room temperature and subjected to shaking for 15 minutes using a Stuart Scientific Flask Shaker Model SF1 set at 500 to 600 oscillations per minute. After extraction the amount of oxycodone dissolved was then determined with detection by HPLC with UV at 206 mm wavelength. The extraction results are presented in Table 2 and Figure 7. The dissolution profiles are determined using the Ph. Eur. Basket Apparatus at 100 rpm at 37°C in 900 ml SGF optionally with 40% ethanol and detection by HPLC with UV at 206 nm wavelength. The dissolution results of Examples 15 to 21 in SGF with 40% ethanol are presented in Table 3 and Figure 8. The dissolution profiles of Examples 15 to 21 in SGF are provided in Table 4 and of Examples 15 to 20 also in Figure 9. The dissolution results in SGF with 40% ethanol and in SGF for Examples 22 to 24 are provided in Tables 5 and 6 and Figures 10 and 11, respectively.

**TABLE 1**

| | **Percent Content (w/w)** | | | | | | | | |
|---|---|---|---|---|---|---|---|---|---|
| **Extruded Product Batch No.** | **Oxycodone HCl** | **Ethylcellulose N10** | **Kollidon SR** | **Avicel PH101** | **Eudragit NE (from NE 40 D)** | **Stearyl Alcohol** | **Stearic Acid** | **Glycerol Dibehenate** | **Total (%)** |
| | | | | | | | | | |
| 15 | 30 | 24.67 | | | 34.66 | 8.67 | | 2 | 100 |
| 16 | 30 | 30 | | | 32 | 6 | | 2 | 100 |
| 17 | 30 | 26 | | | 30 | 12 | | 2 | 100 |
| 18 | 30 | 22 | | | 40 | 6 | | 2 | 100 |
| 19 | 30 | 30 | | | 30 | 8 | | 2 | 100 |
| 20 | 30 | 20 | | | 36 | 12 | | 2 | 100 |
| 21 | 30 | | 26 | | 30 | 12 | | 2 | 100 |
| 22 | 30 | 35 | | | 24 | 9 | | 2 | 100 |
| 23 | 30 | 20 | | 7 | 32 | 9 | | 2 | 100 |
| 24 | 30 | 27 | | | 32 | | 9 | 2 | 100 |

**TABLE 2**

| **Extraction Results for Examples 15 to 21 (40% ethanol at room temperature with shaking for 15 minutes)** | | |
|---|---|---|
| **Example** | **Mean¹⁾ Oxcodone Recovered (mg)** | **Mean¹⁾ Percentage Oxycodone recovered (%)** |
| 15 | 8.3 | 20.8 |
| 16 | 7.7 | 19.3 |
| 17 | 7.6 | 18.9 |
| 18 | 7.5 | 18.8 |
| 19 | 6.6 | 16.4 |
| 20 | 7.1 | 17.8 |
| 21 | 18.8 | 46.9 |

| | | |
|---|---|---|
| 1)Mean values are taken from two runs of the test for each of Examples 15 to 21 | | |

**TABLE 3**

| **Dissolution Results for Examples 15 to 21 in SGF with 40% ethanol** | | | | | | | |
|---|---|---|---|---|---|---|---|
| **Time (hours)** | **% Oxycodone Released¹⁾** | | | | | | |
| | **Ex.15** | **Ex. 16** | **Ex. 17** | **Ex. 18** | **Ex. 19** | **Ex. 20** | **Ex. 21** |
| 0 | 0. | 0 | 0 | 0 | 0 | 0 | 0 |
| 0.5 | 35.80 | 33.43 | 39.21 | 34.78 | 30.64 | 43.04 | 76.65 |
| 1 | 54.08 | 50.60 | 59.54 | 52.55 | 47.51 | 66.22 | 97.08 |
| 1.5 | 64.96 | 60.04 | 70.47 | 63.28 | 57.41 | 77.97 | 97.78 |
| 2 | 73.42 | 68.88 | 78.38 | 71.80 | 65.13 | 85.89 | 97.70 |
| 3 | 85.00 | 81.07 | 89.12 | 84.02 | 77.03 | 94.65 | 98.32 |

| | | | | | | | |
|---|---|---|---|---|---|---|---|
| 1)Mean values are taken from two runs of the test for each of Examples 15 to 21 | | | | | | | |

**TABLE 4**

| **Dissolution Results for Examples 15 to 21 in SGF** | | | | | | | |
|---|---|---|---|---|---|---|---|
| **Time (hours)** | **% Oxycodone Released** | | | | | | |
| | **Ex.15** | **Ex. 16** | **Ex. 17** | **Ex. 18** | **Ex. 19** | **Ex. 20** | **Ex. 21** |
| 0 | 0 | 0 | 0 | 0 | | 0 | 0 |
| 1 | 18.88 | 14.54 | 25.6 | 18.79 | 13.5 | 34.18 | 57.2 |
| 2 | 26.43 | 20.46 | 38.3 | 27.25 | 19.52 | 51.44 | 86.51 |
| 3 | 32.38 | 25.05 | 46.83 | 34.04 | 24.11 | 63.47 | 96.59 |
| 4 | 37.36 | 28.92 | 54.31 | 39.56 | 28.16 | 72.51 | 98.77 |
| 5 | 41.38 | 32.34 | 60.32 | 45.14 | 31.62 | 78.84 | 99.12 |
| 6 | 45.47 | 35.46 | 67.94 | 48.82 | 34.54 | 83.79 | 99.01 |
| 7 | 48.53 | 37.91 | 70.05 | 52.39 | 37.49 | 87.99 | 98.82 |
| 8 | 51.68 | 40.86 | 74.41 | 56.22 | 40.14 | 91.46 | 99.03 |
| 9 | 54.23 | 43.06 | 78.81 | 59.2 | 42.56 | 93.75 | 99.02 |
| 10 | 56.51 | 44.83 | 80.81 | 61 | 44.76 | 95.73 | 98.9 |
| 11 | 59.33 | 47.08 | 83.64 | 64.68 | 47.06 | 96.79 | 98.9 |
| 12 | 61.14 | 48.89 | 85.61 | 68.2 | 48.74 | 97.92 | 99.14 |
| 13 | 63.54 | 50.95 | 88.43 | 70.76 | 51.06 | 98.97 | 98.81 |
| 14 | 65.14 | 52.43 | 89.84 | 71.7 | 52.54 | 98.41 | 98.53 |
| 15 | 66.99 | 53.83 | 92.51 | 72.51 | 54.22 | 98.87 | 98.72 |
| 16 | 68.67 | 55.13 | 94.51 | 77.03 | 55.7 | 98.38 | 98.9 |
| 17 | 70.52 | 56.67 | 93.8 | 78.96 | 57.49 | 99.04 | 99.09 |
| 18 | 73.18 | 58.21 | 96.81 | 79.48 | 58.71 | 98.91 | 98.89 |
| 19 | 74.36 | 59.83 | 96 | 81.41 | 59.88 | 98.89 | 98.77 |
| 20 | 74.78 | 60.41 | 96.94 | 82.94 | 61.24 | 98.65 | 98.67 |
| 21 | 76.73 | 61.74 | 98.68 | 84.18 | 62.72 | 99.13 | 99.09 |
| 22 | 75.76 | 61.26 | 97.7 | 83.56 | 64.19 | 99.17 | 98.81 |
| 23 | 78.34 | 63.46 | 98.07 | 86.81 | 65.06 | 98.92 | 98.51 |
| 24 | 79.53 | 64.69 | 98.16 | 86.55 | 66.13 | 99.07 | 98.66 |

**TABLE 5**

| **Dissolution Results for Examples 22 to 24 in SGF with 40% ethanol** | | | |
|---|---|---|---|
| **Time (hours)** | **% Oxycodone Released¹⁾** | | |
| | **Example 22** | **Example 23** | **Example 24** |
| 0 | 0.00 | 0.00 | 0.00 |
| 0.5 | 31.30 | 37.19 | 36.42 |
| 1 | 45.54 | 55.37 | 52.68 |
| 1.5 | 56.76 | 67.51 | 64.38 |
| 2 | 64.17 | 75.64 | 72.97 |
| 3 | 78.00 | 89.44 | 86.11 |
| 4 | 86.70 | 95.30 | 92.40 |

| | | | |
|---|---|---|---|
| 1)Mean values are taken from two runs of the test for each of Examples 22 to 24 | | | |

**TABLE 6**

| **Dissolution Results for Examples 22 to 24 in SGF** | | | |
|---|---|---|---|
| **Time (hours)** | **% Oxycodone Released** | | |
| | **Example 22** | **Example 23** | **Example 24** |
| 0 | 0.00 | 0.00 | 0.00 |
| 1 | 13.74 | 26.51 | 17.78 |
| 2 | 19.69 | 38.41 | 24.93 |
| 3 | 23.65 | 46.41 | 30.09 |
| 4 | 28.11 | 54.57 | 34.76 |
| 5 | 31.24 | 60.16 | 38.05 |
| 6 | 34.13 | 65.26 | 40.83 |
| 7 | 37.03 | 68.13 | 44.12 |
| 8 | 39.44 | 72.59 | 46.91 |
| 9 | 42.10 | 75.59 | 48.75 |
| 10 | 43.90 | 77.98 | 51.41 |
| 11 | 46.19 | 80.38 | 53.71 |
| 12 | 48.07 | 82.83 | 55.40 |
| 13 | 50.14 | 86.18 | 57.33 |
| 14 | 51.83 | 86.98 | 59.20 |
| 15 | 53.49 | 88.72 | 60.43 |
| 16 | 55.29 | 90.37 | 61.72 |
| 17 | 57.05 | 91.94 | 64.22 |
| 18 | 57.92 | 91.96 | 64.85 |
| 19 | 59.25 | 93.98 | 65.38 |
| 20 | 61.25 | 94.20 | 67.30 |
| 21 | 61.78 | 95.19 | 68.81 |
| 22 | 63.75 | 96.57 | 70.63 |
| 23 | 64.30 | 96.91 | 71.91 |
| 24 | 65.85 | 97.28 | 72.65 |

The present application further pertains to the following numbered embodiments:
1. Use of a sparingly water permeable thermoplastic polymer or a hydrophobic polymer as controlled release matrix material in the manufacture of an opioid controlled release matrix formulation to impart resistance to alcohol extraction of the opioid, wherein said formulation having the sparingly water permeable thermoplastic polymer or hydrophobic polymer as controlled release matrix material releases less opioid in an alcohol extraction test compared to the same formulation but with the sparingly water permeable thermoplastic polymer or hydrophobic polymer substituted entirely or partly by other matrix materials.
2. Use of a sparingly water permeable thermoplastic polymer as controlled release matrix material in the manufacture of an opioid salt controlled release matrix formulation to impart resistance to alcohol extraction of the opioid salt, wherein said formulation after 15 minutes shaking in 40 % ethanol at room temperature releases less than 35 % of opioid salt.
3. Use according to embodiment 2, wherein said formulation releases less than 30 %, more preferred less than 25 % of opioid salt, or from 15 to 25 % opioid salt.
4. Use of a hydrophobic material as controlled release matrix material in the manufacture of an opioid salt controlled release matrix formulation to impart resistance to alcohol extraction of the opioid salt, wherein less than 25 % of the opioid salt is released after 1 hour of in-vitro dissolution of a dosage form comprising said formulation in 900 ml of Simulated Gastric Fluid with 20 % ethanol using USP Apparatus I (basket) operating at 100 rpm at 37 °C.
5. Use of a hydrophobic material as controlled release matrix material in the manufacture of an opioid salt controlled release matrix formulation to impart resistance to alcohol extraction of the opioid salt, wherein less than 25% of the opioid salt is released after 1 hour of in-vitro dissolution of a dosage form comprising said formulation in 500 ml of Simulated Gastric Fluid with 20% ethanol using USP Apparatus I (basket) operating at 100 rpm at 37 °C.
6. Use according embodiment 4 or 5, wherein less than 20% opioid salt, more preferred less than 10% opioid salt, even more preferred less than 5% opioid salt or between 10% and 25% opioid salt is released after 1 hour.
7. Use of a hydrophobic material as controlled release matrix material in the manufacture of an opioid salt controlled release matrix formulation to impart resistance to alcohol extraction of the opioid salt, wherein the ratio of the amount of opioid salt released after 1 hour of in-vitro dissolution of the dosage form comprising said formulation in 900 ml of Simulated Gastric Fluid with 20% ethanol using a USP Apparatus I (basket) apparatus at 100 rpm at 37 °C, to the amount of opioid salt released after 1 hour of in-vitro dissolution of the dosage form comprising said formulation in 900 ml of Simulated Gastric Fluid with 0% ethanol using an USP Apparatus I (basket) apparatus at 100 rpm at 37 °C, is less than about 2:1.
8. Use of a hydrophobic material as controlled release matrix material in the manufacture of an opioid salt controlled release matrix formulation to impart resistance to alcohol extraction of the opioid salt, wherein the ratio of the amount of opioid salt released after 1 hour of in-vitro dissolution of the dosage form comprising said formulation in 500 ml of Simulated Gastric Fluid with 20% ethanol using a USP Apparatus I (basket) apparatus at 100 rpm at 37 °C, to the amount of opioid salt released after 1 hour of in-vitro dissolution of the dosage form comprising said formulation in 500 ml of Simulated Gastric Fluid with 0% ethanol using an USP Apparatus I (basket) apparatus at 100 rpm at 37 °C, is less than about 2:1.
9. Use according to embodiment 7 or 8, wherein the ratio is less than 1.5:1, preferably less than 1:1.
10. Use according to any one of the preceding embodiments, wherein the hydrophobic material or the sparingly permeable thermoplastic polymer is an alkyl cellulose.
11. Use according to embodiment 10, wherein the alkyl cellulose is ethyl cellulose.
12. Use according to any one of the preceding embodiments, wherein the opioid salt is selected from opioid agonists, opioid antagonists in combination with opioid agonists the combination providing an analgesic effect, and mixed opioid agonist/antagonists partial opioid agonists or mixtures thereof in the form of the pharmaceutically acceptable salts thereof.
13. Use according to any one of the preceding embodiments, wherein the opioid salt is selected from alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, in the form of pharmaceutically acceptable salts thereof; and mixtures of any of the foregoing, and the like, preferably from pharmaceutically acceptable salts of any of codeine, morphine, oxycodone, hydrocodone, hydromorphone, or oxymorphone.
14. Use according to any one of the preceding embodiments, wherein the opioid salt is a combination of an opioid agonist salt and an opioid antagonist salt, the combination providing an analgesic effect, wherein the opioid antagonist is selected form the group of naloxone, naltrexone and nalorphine in the form of pharmaceutically acceptable salts thereof.
15. Use according to embodiments 10 to 14, wherein the alkyl cellulose, preferably ethyl cellulose, is used in an amount from 5 to 60% (by wt) of the matrix formulation, preferably from 10 to 50 % (by wt), most preferably from 20 to 45% (by wt) of the matrix formulation, or in an amount of at least 40% (by wt), at least 45% (by wt), at least 50% (by wt), at least 55% (by wt) or at least 60% (by wt) of the matrix formulation.
16. Use according to any one of the preceding embodiments, wherein the ethyl cellulose is combined with at least a second controlled release matrix material selected from a polymethacrylate polymer, preferably a neutral water-insoluble poly (ethyl acrylate, methyl methacrylate) copolymer.
17. Use according to embodiment 16, wherein the polymethacrylate polymer, preferably the neutral water-insoluble poly(ethyl acrylate, methyl acrylate) copolymer is used in an amount of 5% to 66% (by wt), preferably 15% to 50% (by wt), more preferred 20% to 45% (by wt) and most preferred 25% to 45% (by wt) or in a weight amount of at least 5% (by wt), at least 10% (by wt), at least 15% (by wt), at least 20% (by wt) or at least 25% (by wt) of the matrix formulation.
18. Use according to embodiments 10 to 17, wherein the opioid salt is oxycodone hydrochloride or hydromorphone hydrochloride.
19. Use according to any one of the previous embodiments, wherein at least one binder, preferably hydroxy alkyl cellulose, is included in the matrix formulation.
20. Use according to embodiments 10 to 14, wherein the amount of the alkyl cellulose, preferably ethyl cellulose, is less than 20% (by wt), preferably less than 15% (by wt), most preferred less than 10% (by wt) of the matrix formulation.
21. Use according to embodiment 20, wherein the alkylcellulose, preferable ethylcellulose is combined with at least one plasticizer or second controlled release matrix material selected from C₁₂ to C₃₆ aliphatic alcohols or corresponding aliphatic acids, preferably stearyl alcohol, cetyl alcohol, cetostearyl alcohol, stearic acid or palmitic acid or mixtures thereof.
22. Use according to embodiment 21, wherein the alkyl cellulose, preferably the amount of C₁₂ to C₃₆ aliphatic alcohol is at least 5 %, more preferred at least 10 % (by wt), more preferred at least 15% (by wt) and most preferred 20% to 25% (by wt) of the matrix formulation.
23. Use according to embodiments 20 to 22, wherein the opioid salt is a mixture of oxycodone hydrochloride and naloxone hydrochloride in an amount ratio of 2:1.
24. Use according to embodiment 19 to 23, wherein the matrix formulation does not comprise a neutral water-insoluble poly (ethyl acrylate methyl acrylate) copolymer.
25. Use according to any one of the preceding embodiments, wherein the matrix formulation does not comprise a poly(meth)acrylate trimethylammoniummethylacrylate chloride copolymer.
26. Use according to any one of the preceding embodiments, wherein the matrix formulations is prepared in a melt extrusion process.
27. Use according to any of the preceding embodiments wherein the controlled release matrix formulations after 15 minutes shaking in water at room temperature releases less than 15%, less than 10% of opioid salt, preferably less than 7.5% opioid salt, more preferably less than 5% opioid salt.
28. Use according to any of the preceding embodiments wherein the controlled release matrix formulation after 5 minutes standing in water at 50°C followed by 15 minutes shaking at the same temperature releases less than 20% opioid salt, preferably less than 15% opioid salt, more preferably less than 12% opioid salt.
29. Use according to any of the preceding embodiments wherein the controlled release matrix formulation after 5 minutes standing at 75°C followed by 15 minutes shaking at the same temperature releases less than 25% of opioid salt, preferably less than 20% of opioid salt, more preferably less than 15% of opioid salt.
30. Use according to any of the preceding embodiments wherein the controlled release matrix formulation after 5 minutes standing at 100°C followed by 15 minutes shaking at the same temperature releases less than 30% opioid salt, preferably less than 25% opioid salt, more preferably less than 20% opioid salts.
31. Use according to any one of the preceding embodiments wherein the ratio of the weight% amount of the opioid salt released at 50°C, 120 minutes shaking of the controlled release matrix formulation to the weight% amount of opioid salt released at RT 120 minutes shaking of the controlled release matrix formulation is 1.2 or less, preferably 1 or less or 0.9 or less.
32. Use according to any one of the preceding embodiments wherein the ratio of the weight% amount of the opioid salt released at 75°C, 15 minutes shaking of the controlled release matrix formulation to the weight% amount of opioid analgesic released at RT 15 minutes shaking of the controlled release matrix formulation is 1.2 or less, preferably 1 or less or 0.9 or less.
33. Use according to any one of the preceding embodiments wherein the ratio of the weight% amount of the opioid salt released at 100°C, 15 minutes shaking of the controlled release matrix formulation to the weight% amount of opioid salt released at RT 15 minutes shaking of the controlled release matrix formulation is 1.3 or less, preferably 1.2 or less or 0.9 or less.
34. Use according to any one of the preceding embodiments wherein the ratio of the weight% amount of the opioid salt released at 100°C, 120 minutes shaking of the controlled release matrix formulation to the weight% amount of opioid salt released at RT 120 minutes shaking of the controlled release matrix formulation is less than 2, preferably 1.5 or less or 1 or less or 0.9 or less.
35. Use according to any one of the preceding embodiments wherein the controlled release matrix formulation after grinding in a mortar and pestle with 24 rotations of the pestle and extracting in 900 ml water at 37°C for 45 minutes less than 12.5% opioid salt, preferably less than 10% opioid salt, more preferably less than 7.5% opioid salt are released.
36. Use according to any one of the preceding embodiments wherein the controlled release matrix formulation after crushing between two spoons or in a pill crusher and extracting in 2 ml water heated to boiling on a spoon less than 27.5% opioid salt, preferably less than 15% opioid salt, more preferably less than 5% opioid salt are released.
37. A controlled release dosage form comprising:
   a matrix comprising a pharmaceutically acceptable salt of an opioid analgesic in a controlled release material;
   wherein less than 25% of the opioid salt is released after 1 hour of in-vitro dissolution of the dosage form in 900 ml of Simulated Gastric Fluid with 20% ethanol using a USP Apparatus I (basket) apparatus at 100 rpm at 37°C.
38. A controlled release dosage form comprising:
   a matrix comprising a pharmaceutically acceptable salt of an opioid analgesic in a controlled release material;
   wherein less than 25% of the opioid salt is released after 1 hour of in-vitro dissolution of the dosage form in 500 ml of Simulated Gastric Fluid with 20% ethanol using a USP Apparatus I (basket) apparatus at 100 rpm at 37°C.
39. The dosage form of embodiment 37 or 38 comprising a plurality of matrices comprising a pharmaceutically acceptable salt of an opioid analgesic in a controlled release material.
40. The dosage form of embodiment 37 or 38 comprising
   a matrix comprising a pharmaceutically acceptable salt of an opioid analgesic in a pharmaceutically acceptable excipient; and
   a layer comprising a controlled release material disposed about the matrix.
41. The dosage form of embodiment 37 or 38 comprising
   a plurality of matrices comprising a pharmaceutically acceptable salt of an opioid analgesic in a pharmaceutically acceptable excipient; and
   a layer comprising a controlled release material disposed about each of the matrices.
42. The dosage form of any of embodiments 37 to 41, wherein the controlled release material is a hydrophobic material.
43. The dosage form of embodiment 42, wherein the hydrophobic material is ethylcellulose.
44. The dosage form of embodiment 43, wherein ethyl cellulose in a weight amount of at least 40%, at least 45%, at least 50%, at least 55% or at least 60% of the matrix or matrices.
45. The dosage form of embodiment 44, wherein the ethyl cellulose is in a weight amount of at most 70%, at most 80% or at most 90% of the matrix or matrices.
46. The dosage form of embodiment 44, wherein the controlled release material further comprises a polymethacrylate polymer in a weight amount of at least 5%, at least 10%, at least 15%, at least 20% or at least 25% of the matrix or matrices.
47. The dosage form of embodiment 46, wherein the polymethacrylate polymer is in a weight amount of at most 25% or at most 30%, or at most 35% of the matrix or matrices.
48. The dosage form according to embodiments 37 to 47, wherein the matrix or matrices do not contain a water-insoluble neutral poly (ethylacrylate methyl methacrylate) copolymer.
49. The dosage form of any of embodiments 37 to 38, wherein the dosage form further comprises a binder in a weight amount of at least 1%, at least 3%, or at least 5% of the matrix or matrices.
50. The dosage form of embodiment 49, wherein the binder is in a weight amount of at most 7%, or at most 10% of the matrix or matrices.
51. The dosage form of embodiment 49 or 50, wherein the binder is a hydroxyalkylcellulose.
52. The dosage form of any of embodiments 37 to 51, wherein the dosage form further comprises a plasticizer in a weight amount of at least 5%, at least 15%, or at least 25% of the matrix or matrices.
53. The dosage form of embodiment 52, wherein the plasticizer is in a weight amount of at most 30%, or at most 40% of the matrix or matrices.
54. The dosage form of embodiment 52 or 53, wherein the plasticizer has a melting point of at least 80°C.
55. The dosage form of embodiment 54, wherein the plasticizer is hydrogenated castor oil.
56. The dosage form of embodiment 42, wherein the hydrophobic material is an enteric polymer.
57. The dosage form of any of embodiments 37-41, wherein the matrix or matrices are extruded.
58. The dosage form of embodiments 37 or 39, wherein the matrix is a compressed granulation.
59. The dosage form of any of embodiments 37 to 41, wherein the dosage form releases less than 20% opioid salt after 1 hour of in-vitro dissolution of the dosage form in 900 ml of Simulated Gastric Fluid with 20% ethanol using a USP Apparatus I (basket) apparatus at 100 rpm at 37°C.
60. The dosage form of any of embodiments 37 to 41, wherein the dosage form releases more than 5% or more than 10% opioid salt after 1 hour of in-vitro dissolution of the dosage form in 900 ml of Simulated Gastric Fluid with 20% ethanol using a USP Apparatus I (basket) apparatus at 100 rpm at 37°C.
61. The dosage form of any of embodiments 37 to 60, wherein the opioid salt is hydromorphone hydrochloride, and the dosage form comprises:
   2 mg hydromorphone hydrochloride,
   4 mg hydromorphone hydrochloride,
   8 mg hydromorphone hydrochloride,
   12 mg hydromorphone hydrochloride,
   16 mg hydromorphone hydrochloride,
   24 mg hydromorphone hydrochloride,
   32 mg hydromorphone hydrochloride,
   48 mg hydromorphone hydrochloride or
   64 mg hydromorphone hydrochloride.
62. The dosage form of any of embodiments 37 to 60, wherein the opioid salt is oxycodone hydrochloride and the dosage form comprises:
   5 mg oxycodone hydrochloride,
   10 mg oxycodone hydrochloride,
   15 mg oxycodone hydrochloride
   20 mg oxycodone hydrochloride,
   30 mg oxycodone hydrochloride,
   40 mg oxycodone hydrochloride,
   45 mg oxycodone hydrochloride
   60 mg oxycodone hydrochloride,
   80 mg oxycodone hydrochloride,
   90 mg oxycodone hydrochloride
   120 mg oxycodone hydrochloride or
   160 mg oxycodone hydrochloride
63. A method of deterring abuse of an opioid agonist comprising preparing a dosage form according to any of embodiments 37 to 62.
64. A method of manufacturing a controlled release dosage form of any of embodiments 37 to 62 comprising extruding the pharmaceutically acceptable salt of the opioid analgesic and the controlled release material.
65. The method of embodiment 64, comprising cutting the extrudate into a plurality of particles, optionally compressing the particles into a tablet, or
   filling the particles into a pharmaceutically acceptable capsule.
66. A controlled release dosage form comprising an opioid analgesic salt and a controlled release material:
   wherein the ratio of the amount of opioid analgesic salt released after 1 hour of in-vitro dissolution of the dosage form in 500 ml of Simulated Gastric Fluid with 20% ethanol using a USP Apparatus I (basket) apparatus at 100 rpm at 37°C to the amount of opioid analgesic salt released after 1 hour of in-vitro dissolution of the dosage form in 500 ml of Simulated Gastric Fluid with 0% ethanol using a USP Apparatus I (basket) apparatus at 100 rpm at 37°C is less than about 2:1.
67. A controlled release dosage form comprising an opioid analgesic salt and a controlled release material:
   wherein the ratio of the amount of opioid analgesic salt released after 1 hour of in-vitro dissolution of the dosage form in 900 ml of Simulated Gastric Fluid with 20% ethanol using a USP Apparatus I (basket) apparatus at 100 rpm at 37°C to the amount of opioid analgesic salt released after 1 hour of in-vitro dissolution of the dosage form in 900 ml of Simulated Gastric Fluid with 0% ethanol using a USP Apparatus I (basket) apparatus at 100 rpm at 37°C is less than about 2:1.
68. The dosage form of embodiment 66 or 67, comprising a matrix comprising the opioid analgesic salt and the controlled release material.
69. The dosage form of embodiment 66 or 67, comprising a plurality of matrices comprising the opioid analgesic salt and the controlled release material.
70. A controlled release dosage form of embodiment 68 or 69, wherein the opioid analgesic salt is not a combination of oxycodone salt and naloxone salt wherein the matrix comprises ethyl cellulose and stearyl alcohol.
71. The dosage form of embodiment 66 or 67, comprising a matrix comprising the opioid analgesic salt and a pharmaceutically acceptable excipient; and a layer comprising a controlled release material disposed about the matrix.
72. The dosage form of embodiment 66 or 67, comprising a plurality of matrices comprising the opioid analgesic and a pharmaceutically acceptable excipient; and a layer comprising a controlled release material disposed about each of the matrices.
73. The dosage form of any of embodiments 66 to 72, wherein the controlled release material is a hydrophobic material.
74. The dosage form of embodiment 73, wherein the hydrophobic material is ethyl cellulose.
75. The dosage form of embodiment 74, wherein ethyl cellulose in a weight amount of at least 40 %, at least 45 %, at least 50 %, at least 55 % or at least 60 % of the matrix or matrices.
76. The dosage form of embodiment 75, wherein the ethyl cellulose is in a weight amount of at most 70 %, at most 80 % or at most 90 % of the matrix or matrices.
77. The dosage form of embodiment 76, wherein the controlled release material further comprises a polymethacrylate polymer in a weight amount of at least 5 %, at least 10 %, at least 15 %, at least 20 % or at least 25 % of the matrix or matrices.
78. The dosage form of embodiment 77, wherein the polymethacrylate polymer is in a weight amount of at most 30 %, or at most 35 % of the matrix or matrices.
79. The dosage form of any of embodiments 37 to 78, wherein the dosage form further comprises a binder in a weight amount of at least 1 %, at least 3 %, or at least 5 % of the matrix or matrices.
80. The dosage form of embodiment 79, wherein the binder is in a weight amount of at most 7 %, or at most 10 % of the matrix or matrices.
81. The dosage form of embodiment 79 or 80, wherein the binder is a hydroxyalkyl cellulose.
82. The dosage form of any of embodiments 37 to 81, wherein the dosage form further comprises a plasticizer in a weight amount of at least 5 %, at least 15 %, or at least 25 % of the matrix or matrices.
83. The dosage form of embodiment 82, wherein the plasticizer is in a weight amount of at most 30 %, or at most 40 % of the matrix or matrices.
84. The dosage form of embodiment 82 or 83, wherein the plasticizer which has a melting point of at least 80°C.
85. The dosage form of embodiment 84, wherein the plasticizer is hydrogenated castor oil.
86. The dosage form of embodiment 73 wherein the hydrophobic material is an enteric polymer.
87. The dosage form of any of embodiments 66 to 72 wherein the matrix or matrices are extruded.
88. The dosage form of embodiments 66 to 72, wherein the matrix is a compressed granulation.
89. The dosage form of any of embodiments 66 to 72, wherein the ratio of the amount of opioid analgesic released after 1 hour of in-vitro dissolution of the dosage form in 500 ml of Simulated Gastric Fluid with 20 % ethanol using a USP Apparatus I (basket) apparatus at 100 rpm at 37°C to the amount of opioid analgesic released after 1 hour of in-vitro dissolution of the dosage form in 500 ml of Simulated Gastric Fluid with 0% ethanol using a USP Apparatus I (basket) apparatus at 100 rpm at 37°C is less than about 1.5: 1 or less than about 1:1.
90. The dosage form of any of embodiments 37 to 89, wherein the opioid salt is hydromorphone hydrochloride, and comprises:
   2 mg hydromorphone hydrochloride,
   4 mg hydromorphone hydrochloride,
   8 mg hydromorphone hydrochloride,
   12 mg hydromorphone hydrochloride,
   16 mg hydromorphone hydrochloride,
   24 mg hydromorphone hydrochloride,
   32 mg hydromorphone hydrochloride,
   48 mg hydromorphone hydrochloride, or
   64 mg hydromorphone hydrochloride.
91. The dosage form of any of embodiments 37 to 89, wherein the opioid salt is oxycodone hydrochloride, and comprises:
   5 mg oxycodone hydrochloride,
   10 mg oxycodone hydrochloride,
   15 mg oxycodone hydrochloride,
   20 mg oxycodone hydrochloride,
   30 mg oxycodone hydrochloride,
   40 mg oxycodone hydrochloride,
   45 mg oxycodone hydrochloride,
   60 mg oxycodone hydrochloride,
   80 mg oxycodone hydrochloride,
   90 mg oxycodone hydrochloride,
   120 mg oxycodone hydrochloride, or
   160 mg oxycodone hydrochloride.
92. A controlled release dosage form comprising a plurality of matrices comprising a therapeutically effective amount of a pharmaceutically acceptable salt of hydromorphone dispersed in a controlled release material;
   wherein the ratio of the amount of the pharmaceutically acceptable salt of hydromorphone released after 1 hour of in-vitro dissolution of the dosage form in 500 ml of Simulated Gastric Fluid with 20 % ethanol using a USP Apparatus I (basket) apparatus at 100 rpm at 37°C to the amount of a pharmaceutically acceptable salt of hydromorphone released after 1 hour of in-vitro dissolution of the dosage form in 500 ml of Simulated Gastric Fluid with 0 % ethanol using a USP Apparatus I (basket) apparatus at 100 rpm at 37°C is less than about 2:1.
93. The dosage form of embodiment 92 comprising a plurality of extruded matrices comprising a therapeutically effective amount of a pharmaceutically acceptable salt of hydromorphone dispersed in an alkylcellulose.
94. The dosage form of embodiment 92 comprising a plurality of extruded matrices comprising a therapeutically effective amount of a pharmaceutically acceptable salt of hydromorphone dispersed in an ethylcellulose.
95. The dosage form of embodiment 92 comprising a plurality of extruded matrices comprising a therapeutically effective amount of a pharmaceutically acceptable salt ofhydromorphone dispersed in an alkylcellulose, the alkylcellulose being at least 50 %, w/w of the matrices.
96. The dosage form of embodiment 92 comprising a plurality of extruded matrices consisting essentially of a pharmaceutically acceptable salt of hydromorphone dispersed in an alkylcellulose.
97. The dosage form of embodiment 92 comprising a plurality of extruded matrices consisting essentially of a pharmaceutically acceptable salt of hydromorphone dispersed in an alkylcellulose, an optional binder, and an optional plasticizer.
98. The dosage form of embodiment 92 comprising a plurality of extruded matrices comprising a pharmaceutically acceptable salt of hydromorphone dispersed in an alkylcellulose, wherein the matrices do not comprise an acrylic polymer.
99. The dosage form of any of embodiments 27 to 88 comprising a controlled release matrix formulation which does not contain more than 15% (by wt) preferably more than 20 % (by wt) C₁₂ to C₃₆ aliphatic alcohol selected from the group consisting of stearyl alcohol, cetyl alcohol and cetostearyl alcohol.
100. The dosage form according to any of the preceding embodiments wherein the dosage form after 15 minutes shaking in water at room temperature releases less than 15%, less than 10% of opioid salt, preferably less than 7.5% opioid salt, more preferably less than 5% opioid salt.
101. The dosage form according to any of the preceding embodiments wherein the dosage form after 5 minutes standing in water at 50°C followed by 15 minutes shaking at the same temperature releases less than 20% opioid salt, preferably less than 15% opioid salt, more preferably less than 12% opioid salt.
102. The dosage form according to any of the preceding embodiments wherein the dosage form after 5 minutes standing at 75°C followed by 15 minutes shaking at the same temperature releases less than 25% of opioid salt, preferably less than 20% of opioid salt, more preferably less than 15% of opioid salt.
103. The dosage form according to any of the preceding embodiments wherein the dosage form after 5 minutes standing at 100°C followed by 15 minutes shaking at the same temperature releases less than 30% opioid salt, preferably less than 25% opioid salt, more preferably less than 20% opioid sal.
104. The dosage form according to any one of the preceding embodiments wherein the ratio of the weight% amount of the opioid salt released at 50°C, 120 minutes shaking of the dosage form to the weight% amount of opioid salt released at RT 120 minutes shaking of the dosage form is 1.2 or less, preferably 1 or less or 0.9 or less.
105. The dosage form according to any one of the preceding embodiments wherein the ratio of the weight% amount of the opioid salt released at 75°C, 15 minutes shaking of the dosage form to the weight% amount of opioid analgesic released at RT 15 minutes shaking of the dosage form is 1.2 or less, preferably 1 or less or 0.9 or less.
106. The dosage form according to any one of the preceding embodiments wherein the ratio of the weight% amount of the opioid salt released at 100°C, 15 minutes shaking of the dosage form to the weight% amount of opioid salt released at RT 15 minutes shaking of the dosage form is 1.3 or less, preferably 1.2 or less or 0.9 or less.
107. The dosage form according to any one of the preceding embodiments wherein the ratio of the weight% amount of the opioid salt released at 100°C, 120 minutes shaking of the dosage form to the weight% amount of opioid salt released at RT 120 minutes shaking of the dosage form is less than 2, preferably 1.5 or less, 1 or less or 0,9 or less.
108. The dosage form according to any one of the preceding embodiments wherein after grinding in a mortar and pestle with 24 rotations of the pestle and extracting in 900 ml water at 37°C for 45 minutes less than 12.5% opioid salt, preferably less than 10% opioid salt, more preferably less than 7.5% opioid salt are released.
109. Use according to any one of the preceding embodiments wherein after crushing between two spoons or in a pill crusher and extracting in 2 ml water heated to boiling on a spoon less than 27.5% opioid salt, preferably less than 15% opioid salt, more preferably less than 5% opioid salt are released.
110. Use of a dosage form according to embodiments 37 to 108 in the manufacture of a medicament for the treatment of pain.
111. A method of deterring abuse of an opioid agonist comprising preparing a dosage form according to any of embodiments 37 to 108.
112. A method of manufacturing a controlled release dosage form of any of embodiments 69 to 108 comprising extruding the pharmaceutically acceptable salt of the opioid analgesic and the controlled release material.
113. The method of embodiment 112, comprising cutting the extrudate into a plurality of particles, optionally comprising compressing the particles into a tablet or filling the particles into a pharmaceutically acceptable capsule.

## Claims

1. A controlled release dosage form comprising an opioid analgesic salt and a controlled release material:
wherein the ratio of the amount of opioid analgesic salt released after 1 hour of in-vitro dissolution of the dosage form in 500 ml, or 900 ml, respectively, of Simulated Gastric Fluid with 20% ethanol using a USP Apparatus I (basket) apparatus at 100 rpm at 37 °C to the amount of opioid analgesic salt released after 1 hour of in-vitro dissolution of the dosage form in 500 ml, or 900 ml, respectively, of Simulated Gastric Fluid with 0% ethanol using a USP Apparatus I (basket) apparatus at 100 rpm at 37 °C is less than about 2:1.

2. The dosage form of claim 1, comprising a matrix comprising the opioid analgesic salt and the controlled release material, or comprising a plurality of matrices comprising the opioid analgesic salt and the controlled release material.

3. The controlled release dosage form of claim 2, wherein the opioid analgesic salt is not a combination of oxycodone salt and naloxone salt wherein the matrix comprises ethyl cellulose and stearyl alcohol.

4. The dosage form of claim 1, comprising a matrix comprising the opioid analgesic salt and a pharmaceutically acceptable excipient; and a layer comprising a controlled release material disposed about the matrix.

5. The dosage form of claim 1, comprising a plurality of matrices comprising the opioid analgesic and a pharmaceutically acceptable excipient; and a layer comprising a controlled release material disposed about each of the matrices.

6. A controlled release dosage form comprising:
a matrix comprising a pharmaceutically acceptable salt of an opioid analgesic in a controlled release material;
wherein less than 25% of the opioid salt is released after 1 hour of in-vitro dissolution of the dosage form in 900 ml, or 500 ml, respectively, of Simulated Gastric Fluid with 20 % ethanol using a USP Apparatus I (basket) apparatus at 100 rpm at 37 °C.

7. The dosage form of claim 6 comprising
a plurality of matrices comprising a pharmaceutically acceptable salt of an opioid analgesic in a controlled release material, or
a matrix comprising a pharmaceutically acceptable salt of an opioid analgesic in a pharmaceutically acceptable excipient; and
a layer comprising a controlled release material disposed about the matrix, or
a plurality of matrices comprising a pharmaceutically acceptable salt of an opioid analgesic in a pharmaceutically acceptable excipient; and
a layer comprising a controlled release material disposed about each of the matrices.

8. The dosage form of any of claims 1 or 6, wherein the controlled release material is a hydrophobic material, preferably ethyl cellulose.

9. The dosage form of claim 8, wherein the ethyl cellulose is in a weight amount of at least 40 %, at least 45 %, at least 50 %, at least 55 % or at least 60 % of the matrix or matrices, preferably wherein the ethyl cellulose is in a weight amount of at most 70 %, at most 80 % or at most 90 % of the matrix or matrices.

10. The dosage form of claim 9, wherein the controlled release material further comprises a polymethacrylate polymer in a weight amount of at least 5 %, at least 10 %, at least 15 %, at least 20 % or at least 25 % of the matrix or matrices, preferably wherein the polymethacrylate polymer is in a weight amount of at most 30 %, or at most 35 % of the matrix or matrices.

11. The dosage form of any of claims 1 to 10, wherein the dosage form further comprises a binder in a weight amount of at least 1 %, at least 3 %, or at least 5 % of the matrix or matrices, preferably wherein the binder is in a weight amount of at most 7 %, or at most 10 % of the matrix or matrices.

12. The dosage form of claim 11, wherein the binder is a hydroxyalkyl cellulose.

13. The dosage form of any of claims 1 to 12, wherein the dosage form further comprises a plasticizer in a weight amount of at least 5 %, at least 15 %, or at least 25 % of the matrix or matrices, preferably wherein the plasticizer is in a weight amount of at most 30 %, or at most 40 % of the matrix or matrices.

14. The dosage form of claim 13, wherein the plasticizer which has a melting point of at least 80 °C, preferably wherein the plasticizer is hydrogenated castor oil.

15. The dosage form of claim 8, wherein the hydrophobic material is an enteric polymer.

16. The dosage form of any of claims 1 to 7 wherein the matrix or matrices are extruded.

17. The dosage form of claims 1 to 7, wherein the matrix is a compressed granulation.

18. The dosage form of any of claims 1 to 5, wherein the ratio of the amount of opioid analgesic released after 1 hour of in-vitro dissolution of the dosage form in 500 ml of Simulated Gastric Fluid with 20 % ethanol using a USP Apparatus I (basket) apparatus at 100 rpm at 37 °C to the amount of opioid analgesic released after 1 hour of in-vitro dissolution of the dosage form in 500 ml of Simulated Gastric Fluid with 0% ethanol using a USP Apparatus I (basket) apparatus at 100 rpm at 37 °C is less than about 1.5: 1 or less than about 1:1.

19. The dosage form of any of claims 1 to 17 comprising a controlled release matrix formulation which does not contain more than 15% (by wt) preferably more than 20 % (by wt) C₁₂ to C₃₆ aliphatic alcohol selected from the group consisting of stearyl alcohol, cetyl alcohol and cetostearyl alcohol.

20. The dosage form according to any of the preceding claims wherein the dosage form after 15 minutes shaking in water at room temperature releases less than 15%, less than 10% of opioid salt, preferably less than 7.5% opioid salt, more preferably less than 5% opioid salt.

21. The dosage form according to any of the preceding claims wherein the dosage form after 5 minutes standing in water at 50 °C followed by 15 minutes shaking at the same temperature releases less than 20% opioid salt, preferably less than 15% opioid salt, more preferably less than 12% opioid salt, and/or wherein the dosage form after 5 minutes standing at 75 °C followed by 15 minutes shaking at the same temperature releases less than 25% of opioid salt, preferably less than 20% of opioid salt, more preferably less than 15% of opioid salt, and/or wherein the dosage form after 5 minutes standing at 100 °C followed by 15 minutes shaking at the same temperature releases less than 30% opioid salt, preferably less than 25% opioid salt, more preferably less than 20% opioid salt.

22. The dosage form according to any one of the preceding claims wherein the ratio of the weight% amount of the opioid salt released at 50 °C, 120 minutes shaking of the dosage form to the weight% amount of opioid salt released at RT 120 minutes shaking of the dosage form is 1.2 or less, preferably 1 or less or 0.9 or less, and/or wherein the ratio of the weight% amount of the opioid salt released at 75 °C, 15 minutes shaking of the dosage form to the weight% amount of opioid analgesic released at RT 15 minutes shaking of the dosage form is 1.2 or less, preferably 1 or less or 0.9 or less.

23. The dosage form according to any one of the preceding claims wherein the ratio of the weight% amount of the opioid salt released at 100 °C, 15 minutes shaking of the dosage form to the weight% amount of opioid salt released at RT 15 minutes shaking of the dosage form is 1.3 or less, preferably 1.2 or less or 0.9 or less, and/or wherein the ratio of the weight% amount of the opioid salt released at 100 °C, 120 minutes shaking of the dosage form to the weight% amount of opioid salt released at RT 120 minutes shaking of the dosage form is less than 2, preferably 1.5 or less, 1 or less or 0,9 or less.

24. The dosage form according to any one of the preceding claims wherein after grinding in a mortar and pestle with 24 rotations of the pestle and extracting in 900 ml water at 37 °C for 45 minutes less than 12.5% opioid salt, preferably less than 10% opioid salt, more preferably less than 7.5% opioid salt are released.

25. A controlled release dosage form comprising a plurality of matrices comprising a therapeutically effective amount of a pharmaceutically acceptable salt of hydromorphone dispersed in a controlled release material;
wherein the ratio of the amount of the pharmaceutically acceptable salt of hydromorphone released after 1 hour of in-vitro dissolution of the dosage form in 500 ml of Simulated Gastric Fluid with 20 % ethanol using a USP Apparatus I (basket) apparatus at 100 rpm at 37 °C to the amount of a pharmaceutically acceptable salt of hydromorphone released after 1 hour of in-vitro dissolution of the dosage form in 500 ml of Simulated Gastric Fluid with 0 % ethanol using a USP Apparatus I (basket) apparatus at 100 rpm at 37 °C is less than about 2:1.
